(19) Europäisches Patentamt
European Patent Office
Office européen des brevets

(11) EP 2 543 428 B1

(12) # EUROPEAN PATENT SPECIFICATION

(45) Date of publication and mention of the grant of the patent:
**05.06.2019 Bulletin 2019/23**

(51) Int Cl.:
***A61L 33/00*** *(2006.01)* ***B01D 71/68*** *(2006.01)*

(21) Application number: **12183460.0**

(22) Date of filing: **20.08.2003**

(54) **Modified membrane substrate with reduced platelet adsorption**

Modifiziertes Substrat mit reduzierter Thrombozytadsorption

Substrat modifié à adsorption réduite des plaquettes

(84) Designated Contracting States:
**AT BE BG CH CY CZ DE DK EE ES FI FR GB GR HU IE IT LI LU MC NL PT RO SE SI SK TR**

(30) Priority: **21.08.2002 JP 2002240247**

(43) Date of publication of application:
**09.01.2013 Bulletin 2013/02**

(62) Document number(s) of the earlier application(s) in accordance with Art. 76 EPC:
**03792729.0 / 1 535 657**

(73) Proprietor: **TORAY INDUSTRIES, INC.**
**Tokyo 103 (JP)**

(72) Inventors:
- **UENO, Yoshiyuki**
  **Shiga 520-8558 (JP)**
- **TAKAHASHI, Hiroshi**
  **Otsu-shi, Shiga 520-8558 (JP)**
- **SUGAYA, Hiroyuki**
  **Otsu-shi, Shiga 520-2101 (JP)**

(74) Representative: **Mewburn Ellis LLP**
**City Tower**
**40 Basinghall Street**
**London EC2V 5DE (GB)**

(56) References cited:
**EP-A1- 1 439 212**
**JP-A- 11 047 570**
**JP-A- 53 134 876**
**JP-A- 2000 237 557**
**JP-A- 2000 254 222**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### Technical Field

The present invention relates to a modified substrate wherein the surface thereof is subjected to a hydrophilization treatment. The modified substrate of the present invention can be preferably used in medical devices. Preferably, the modified substrate of the present invention can also be used as, for example, separation membranes for water treatment, separation membranes of biogenic substances, instruments used for biological experiments, bioreactors, molecular motors, drug delivery systems(DDS), protein chips, DNA chips, biosensors, or components of analytical instruments. In particular, the modified substrate of the present invention is preferably used for applications in which the substrate is brought into contact with a biogenic substance, for example, a module for blood purification such as an artificial kidney.

### Background Art

In medical devices that are in contact with a body fluid, for example, an artificial blood vessel, a catheter, a blood bag, a contact lens, an intraocular lens, and an artificial kidney, biocompatibility, in particular, hematologic compatibility is an important problem. For example, in separation membranes used for blood purification, adhesion of proteins, or adhesion or activation of blood platelets causes blood clotting. It is known that performing a hydrophilization treatment on the surface of a substrate is effective in remedying such a problem of hematologic compatibility. For example, polysulfone polymers are used as a material for the separation membranes for blood purification. In order to provide a polysulfone with hematologic compatibility, a hydrophilic polymer such as polyvinylpyrrolidone is mixed in the stock solution for preparation of the membrane. Although this method provides hematologic compatibility to some degree, the hematologic compatibility is not sufficient.

In a method disclosed in Japanese Unexamined Patent Application Publication No. 10-118472, in order to improve hematologic compatibility on the surface of a substrate, a polysulfone separation membrane is brought into contact with a solution of a hydrophilic polymer such as polyvinylpyrrolidone. Thus, the separation membrane physically adsorbs the hydrophilic polymer. However, in this method, the hydrophilic polymer is only adsorbed on the surface. Therefore, when the separation membrane is in contact with blood, the hydrophilic polymer may be dissolved into the blood. In a method disclosed in Japanese Unexamined Patent Application Publication No. 6-238139, a polysulfone separation membrane is brought into contact with a solution of a hydrophilic polymer such as polyvinylpyrrolidone. In this method, an insolubilized hydrophilic polymer layer is formed on the surface of the membrane utilizing radiation crosslinking. This method suppresses the dissolution of the hydrophilic polymer. However, when the membrane is in contact with blood, the insolubilized hydrophilic polymer activates the blood platelets. As a result, hematologic compatibility is deteriorated rather than improved.

JPH 11-047570A discloses a separation membrane by making a polyalkylene glycol exist on at least part of a surface of the membrane consisting of a polysulfone resin containing polyvinyl pyrrolidone. JP 2000-254222A concerns a hollow fiber membrane prepared from a stock solution in which a hydrophilic polymer and a hydrophobic polymer and solvent are mixed in a common solvent and in which the ratio of hydrophilic polymer against the hydrophobic polymer on the outer surface of the membrane is 5 to 25%. In JP 2000-237557A a membrane is dipped in a polyalkylene glycol solution and then irradiated in order to provide a membrane provided with hydrophobic property and free from elution of water-soluble polymer. JP53-134876A proposes a molded product, such as a membrane, consisting of a water-soluble high polymer and vinylidene fluoride polymer which is irradiated with radiation. EP-1439212A discloses membrane substrates obtained from a feedstock of polysulfone and polyvinylpyrrolidone. Hollow fiber membranes are irradiated.

It is an object of the present invention to provide a modified substrate having high hematologic compatibility wherein a hydrophilic polymer is immobilized on the surface of the substrate, and a method for producing the same.

As a result of intensive study, the present inventors have found a method for immobilizing a hydrophilic polymer on a substrate without excessive crosslinking or degrading the hydrophilic polymer, and have accomplished the present invention.

In a first aspect the present invention provides a modified substrate as defined in the accompanying claim 1, wherein the number of adhered human blood platelets in an area of 4.3x103 μm2 is 10 or less.

The present invention also includes a separation membrane using the modified substrate.

Further aspects of the invention concern a medical device comprising the modified substrate or the separation membrane.

### Brief Description of the Drawing

Fig. 1 is a view showing an example of the basic structure of an artificial kidney system.

Best Mode for Carrying Out the Invention

In the present invention, a substrate is irradiated with radiation while the substrate is brought into contact with an aqueous solution of a hydrophilic polymer, thus producing a modified substrate wherein the hydrophilic polymer is immobilized on the surface of the substrate. Hematologic compatibility of a substrate depends on the surface state of areas that are in contact with blood. In general, the higher the hydrophilicity of the surface and the higher the mobility of the hydrophilic polymer immobilized on the surface, the higher the hematologic compatibility of the substrate is. This is because a hydrophilic polymer having high mobility eliminates proteins or blood platelets due to its molecular motion.

Herein, the term immobilization refers to a state in which a hydrophilic polymer is bonded with a substrate. In the present invention, it is necessary for the soluble hydrophilic polymer ratio to be 15 weight percent or less, and preferably, 10 weight percent or less. Herein, the term soluble hydrophilic polymer refers to a hydrophilic polymer that is neither crosslinked nor insolubilized due to immobilization on the substrate. The soluble hydrophilic polymer ratio is defined as a ratio of the soluble hydrophilic polymer to the total of the hydrophilic polymer in the modified substrate. A detailed method for measuring the soluble hydrophilic polymer ratio will be described later. When the soluble hydrophilic polymer ratio exceeds 15 weight percent, bonding of the hydrophilic polymer with the substrate is insufficient. Therefore, when the modified substrate is brought into contact with blood, the hydrophilic polymer may be dissolved into the blood.

The amount of dissolution of the hydrophilic polymer is preferably 0.5 $mg/m^2$ or less, more preferably, 0.3 $mg/m^2$ or less. Herein, the amount of dissolution of the hydrophilic polymer is defined as follows: A substrate is brought into contact with purified water at 37°C for 4 hours. The amount of the hydrophilic polymer that is dissolved into the purified water is converted to an amount per unit area of the measured substrate. A detailed method for measuring the amount of dissolution will be described later. When the amount of dissolution of the hydrophilic polymer exceeds the above range, there is a concern that, in medical devices that are in contact with blood, the dissolved hydrophilic polymer is accumulated in the body of patients. When the molecular weight of the hydrophilic polymer exceeds 50,000, the polymer is not filtered by the kidneys and is not excreted from the body. Therefore, such accumulation is a particular concern. Furthermore, when the substrate is used as an artificial kidney, the artificial kidney is used for patients who have poor or no their renal function. Therefore, even when the molecular weight of the hydrophilic polymer is 50,000 or less, the accumulation in the body of patients is a concern. In addition, when the substrate is used as analytical instruments such as a protein chip or a biosensor, there is a concern that the dissolved hydrophilic polymer becomes an inhibiting factor in the analysis.

The condition for irradiating with radiation is preferably controlled as follows. In an aqueous solution of a hydrophilic polymer being in contact with a substrate, the maximum increasing value of ultraviolet absorption value in the wavelength range of 260 to 300 nm, the increase being caused by irradiating with radiation, is preferably 1 or less, more preferably 0.5 or less. Herein, the maximum increasing value of ultraviolet absorption value is defined as follows: Values are calculated by subtracting the ultraviolet absorption values of the aqueous solution of the hydrophilic polymer in the range of 260 to 300 nm before irradiating with radiation from the ultraviolet absorption values of the aqueous solution of the hydrophilic polymer in the same wavelength range after irradiating with radiation. Among the above values, the maximum value in the above wavelength range is defined as the maximum increasing value of the ultraviolet absorption value. Under some conditions for irradiating with radiation, the hydrophilic polymer is degraded to generate a substance absorbing light in the wavelength range of 260 to 300 nm and having a relatively high reactivity. In particular, in medical devices, the amount of such a substance is preferably small in terms of safety.

In the modified substrate of the present invention, a surface hydrophilic polymer ratio is at least 20 weight percent. Herein, the surface hydrophilic polymer ratio is defined as a ratio represented by A/(A+B), wherein (A) is the weight of the monomer unit of the hydrophilic polymer on the surface of the modified substrate (the number of moles of the monomer unit x the molecular weight of the monomer unit) and (B) is the weight of the monomer unit of the polymer forming the substrate on the surface of the modified substrate (the number of moles of the monomer unit x the molecular weight of the monomer unit). This surface hydrophilic polymer ratio is a parameter representing the degree of hydrophilicity on the surface of the modified substrate.

The surface hydrophilic polymer ratio is measured by analyzing only the surface of the modified substrate, i.e., the depth profile of about 10 nm from the surface, by X-ray photoelectron spectrometry ESCA). The surface hydrophilic polymer ratio is at least 20 weight percent, more preferably, at least 32 weight percent. When the surface hydrophilic polymer ratio is less than 20 weight percent, the effect at suppressing the adhesion of organic matter such as proteins, or biogenic substances is decreased. This is because the hydrophilic polymer cannot cover the surface of the substrate, and therefore, the ratio of the substrate exposed on the surface of the modified substrate is increased.

In the modified substrate of the present invention, a hydrophilic polymer is immobilized on the surface of the substrate, and in addition, for example, excessive crosslinking or degradation of the hydrophilic polymer is prevented. As a result, the adhesion of organic matter such as proteins, or biogenic substances can be suppressed. The modified substrate of the present invention particularly has high hematologic compatability. Specifically, in the modified substrate of the present invention, the number of adhered human blood platelets is $10/4.3x10^3$ μm2 or less. The number of adhered

blood platelets is defined as follows: A modified substrate is brought into contact with blood for one hour. The number of blood platelets adhered on the surface of the modified substrate is represented as the number per $4.3x10^3$ $\mu m^2$ of the surface area of the modified substrate. Detailed methods for measuring the number of adhered blood platelets will be described later. When the number of adhered human blood platelets exceeds $10/4.3\times10^3$ $\mu m^2$, hematologic compatibility is insufficient, and in addition, the effect at suppressing the adhesion of organic matter such as proteins, or biogenic substances is also insufficient.

Because of its high hematologic compatibility, the modified substrate of the present invention can be preferably used as medical substrates. The medical substrates used in the present invention include substrates used in an artificial blood vessel, a catheter, a blood bag, a contact lens, an intraocular lens, auxiliary instruments for surgical operation, and a module for blood purification. In particular, the modified substrate of the present invention is suitable for applications in which the substrate is brought into contact with a biogenic substance, for example, a module for blood purification such as an artificial kidney. Herein, the module for blood purification refers to a module having a function of circulating the blood in order to remove waste products or harmful substances from the blood in order to excrete them from the body. Examples of the module for blood purification include an artificial kidney and an adsorption column for exotoxins. The module for an artificial kidney includes a coil type, a flat plate type, and a hollow fiber membrane type. In terms of, for example, high processing efficiency, the hollow fiber membrane type is preferable.

Furthermore, medical substrates used for adsorbing and removing substances such as a cytokine, e.g., interleukin-6 (hereinafter abbreviated as IL-6), substances having an adverse effect on the living body, are known. Preferably, such medical substrates also have high hematologic compatibility. As a result of hydrophilization treatment performed on the surface of the substrate, the adhesion on the substrate of blood platelets or proteins related to clotting is suppressed. However, at the same time, the adsorption on the substrate of target substances to be removed such as IL-6 is also suppressed. The modified substrate of the present invention can achieve high hematologic compatibility while maintaining the adsorption of a cytokine such as IL-6. Specifically, a modified substrate having high hematologic compatibility can be produced, while the adsorptivity to cytokine of the modified substrate is maintained so as to be at least 90% of the adsorptivity to cytokine of the substrate before modification. In the modified substrate of the present invention, the adsorptivity to IL-6 is preferably at least 0.1 $ng/cm^2$. When the adsorptivity to IL-6 is within this range, the modified substrate can be preferably used as an adsorption column for IL-6.

Preferably, the modified substrate of the present invention can also be used as, for example, separation membranes for water treatment, separation membranes of biogenic substances, instruments used for biological experiments, bioreactors, molecular motors, DDS, protein chips, DNA chips, biosensors, or components of analytical instruments, utilizing the feature in which the modified substrate suppresses the adhesion of biogenic substances.

In addition, since the modified substrate of the present invention includes a hydrophilic polymer having a low degree of three-dimensional crosslinking thereon, the modified substrate can be applied to a material that requires low frictionality.

In the present invention, the substrate represents a material to which hydrophilicity is provided. The substrate is composed of a polymeric material. Examples of polymeric materials include polysulfones, polystyrene, polyurethanes, polycarbonate, polymethylmethacrylate, polyethylene, polypropylene, polyvinylidene fluoride, polyacrylonitrile, polyesters, and polyamides.

In the present invention the substrate is composed of a polymeric material which is a polysulfone or copolymers thereof.

Examples of the form of the substrate include a fiber, a film, a resin, and a separation membrane. The form of the substrate is not limited to the above.

When **a** substrate is used as a medical substrate, **a** substrate is preferably composed of, for example, polyvinyl chloride; cellulose polymers; polystyrene; polymethylmethacrylate; polycarbonate; polysulfone polymers such as polysulfones and polyethersulfones; polyurethanes; polyacrylonitrile; and polyvinylidene fluoride. In
**the present invention** polysulfone polymers are selected because polysulfone polymers are readily formed and separation membranes composed of polysulfone polymers have an excellent performance in terms of the permeation of a substance.

Polysulfone polymers include aromatic rings, a sulfonyl group, and an ether group in the main chain. For example, polysulfones represented by the following chemical formula (1) and/or (2) are preferably used. Symbol n in the formulae is preferably 50 to 80.

(1)

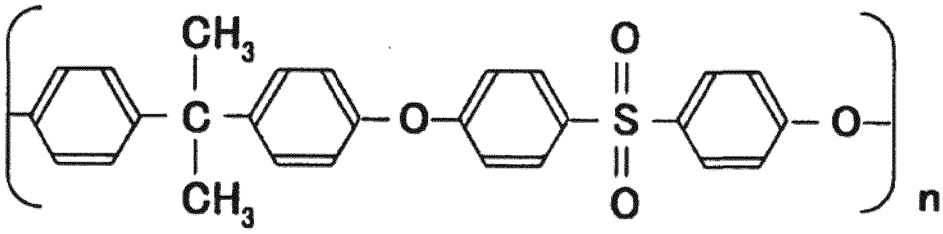

(2)

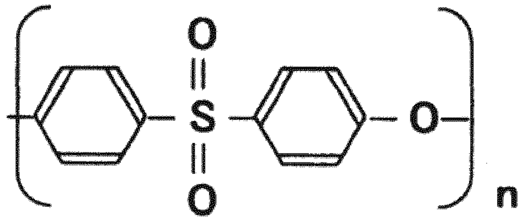

Examples of the polysulfones include Udel (registered trademark) polysulfone P-1700, P-3500 (from Teijin Amoco Engineering Plastics Limited); Ultrason (registered trademark) S3010 and S6010 (from BASF); Victrex (registered trademark) (from Sumitomo Chemical Co., Ltd.); Radel (registered trademark) A (from Teijin Amoco Engineering Plastics Limited); and Ultrason (registered trademark) E (from BASF). Although the polysulfones used in the present invention preferably include only the repeating unit represented by the above formula (1) and/or (2), the polysulfones may be copolymerized with other monomers so long as the advantage of the present invention is not impaired. The amount of the other copolymerization monomers is preferably 10 weight percent or less.

When the substrate is used as a medical substrate for adsorbing and removing a cytokine such as IL-6, the substrate is preferably composed of a hydrophobic polymer because such a polymer has a high adsorbing performance. Because of its high adsorbing performance, polymethylmethacrylate is particularly preferable.

In the present invention, a hydrophilic polymer refers to a polymer including a hydrophilic functional group in the main chain or the side chain of the polymer. Hydrophilic polymers having solubility in water at 25°C of, preferably, at least 0.001 weight percent, more preferably, at least 0.01 weight percent, and most preferably, at least 0.1 weight percent, are readily applied to the present technology. Examples of a hydrophilic polymer include polyvinylpyrrolidone, polyethylene glycol, polypropylene glycol, polyvinyl alcohol, polyethyleneimine, polyallylamines, polyvinylamine, polyvinyl acetate, polyacrylic acid, polyacrylamide, and copolymers and graft polymers of these and other monomers. Nonionic hydrophilic polymers such as polyalkylene glycols and polyvinylpyrrolidone provide an inhibiting effect of nonspecific adsorption.

**In the present invention**, polyvinylpyrrolidone is selected because it provides a high inhibiting effect of adsorption.

The method for measuring the amount of hydrophilic polymer immobilized on the surface of the substrate is different depending on the kinds of substrate and hydrophilic polymer and the method is appropriately selected. Preferably, the amount of the hydrophilic polymer bonded on the modified substrate is directly measured. However, more simple methods may also be used. For example, the concentration of the hydrophilic polymer in an aqueous solution before irradiating with radiation may be compared with that in the aqueous solution after irradiating with radiation. Thus, the amount of decrease in the hydrophilic polymer in the aqueous solution is calculated. This amount may be defined as the amount of the immobilized hydrophilic polymer. In another simple method, the contact angle of the surface may be measured to estimate the amount of the immobilized hydrophilic polymer.

The molecular weight of the hydrophilic polymer is preferably at least 100, more preferably, at least 500, and most preferably at least 1,000. The molecular weight of the hydrophilic polymer is preferably 50,000 or less.

Examples of the radiation used include $\alpha$-ray, $\beta$-ray, $\gamma$-ray, X-ray, ultraviolet rays, and electron beams. Medical devices such as an artificial kidney require sterilization. In terms of low residual toxicity and convenience, recently, radiosterilization using $\gamma$-ray or an electron beam is often used. In other words, when the method of the present invention is used in medical substrates, sterilization and modification of a substrate can be preferably achieved at the same time. In particular, the method of the present invention is preferably applied to an artificial kidney. In the artificial kidney, a wet type is mainly used in which the separation membrane is in a state containing water. Accordingly, the method of the present invention can be conveniently used by only replacing the water with an aqueous solution containing a hydrophilic polymer solution.

When sterilization and modification of a substrate are performed at the same time, the substrate is irradiated with radiation with an absorbed dose of at least 20 kGy. This is because an absorbed dose of at least 20 kGy is effective

in order to sterilize, for example, a module for blood purification with γ-ray. However, when the absorbed dose is 20 kGy or more, the hydrophilic polymer is subjected to three-dimensional crosslinking or degraded, thereby decreasing hematologic compatibility. Therefore, in the present invention, an antioxidant is added. Specifically, the substrate is irradiated with radiation while the substrate is brought into contact with an aqueous solution containing a **hydrophilic** polymer and an antioxidant. The addition of the antioxidant provides the following features: Excessive crosslinking or degradation of the hydrophilic polymer can be prevented, while the hydrophilic polymer is immobilized, furthermore, sterilization can be performed at the same time.

The antioxidant according to the present invention refers to molecules that readily provide other molecules with electrons. When a hydrophilic polymer such as polyvinylpyrrolidone is subjected to a radical reaction with radiation, the antioxidant inhibits the reaction. Examples of **an** antioxidant include water-soluble vitamins such as vitamin C; polyphenols; alcohols such as methanol, ethanol, propanol, ethylene glycol, and glycerin; saccharides such as glucose, galactose, mannose, and trehalose; inorganic salts such as sodium hydrosulfite, sodium pyrosulfite, and sodium dithionate; uric acid; cysteine; glutathione; and oxygen. These antioxidants may be used alone or in combination of two or more. When the method of the present invention is used in medical devices, the safety must be considered. Therefore, antioxidants having low toxicity are **used in such a case. In the present invention**, alcohols, saccharides, and inorganic salts are used.

The concentration of antioxidant in an aqueous solution is different depending on, for example, the kind of antioxidant and the exposure dose of radiation. An excessively low concentration of antioxidant causes three-dimensional crosslinking or degradation of the hydrophilic polymer to decrease hematologic compatibility. On the other hand, the addition of an excessive amount of antioxidant decreases the immobilization efficiency on the substrate. Therefore, sufficient hematologic compatibility is not achieved.

A method for producing a modified substrate of the present invention will now be described in detail with reference to an example using an antioxidant.

In a method for modifying the substrate, the substrate is irradiated with radiation while the substrate is brought into contact with an aqueous solution containing a hydrophilic polymer and an antioxidant. For example, when the substrate is a film, preferably, the substrate is irradiated with radiation while the substrate is immersed in an aqueous solution containing a hydrophilic polymer and an antioxidant. When the substrate is a hollow substrate such as a hollow fiber membrane and hydrophilicity should be provided on the inner surface of the hollow part, the aqueous solution is filled inside of the hollow part and then the substrate is preferably irradiated with radiation. Furthermore, when the substrate is disposed in a module, the aqueous solution is filled in the module and then the whole module is preferably irradiated with radiation. For example, in an artificial kidney, separation membranes are disposed in a module case. In such a case, an aqueous solution containing a hydrophilic polymer and an antioxidant is filled in the module and then the whole module may be irradiated with radiation. Alternatively, only the separation membranes may be irradiated with radiation while the separation membranes are immersed in the aqueous solution containing the hydrophilic polymer and the antioxidant. Subsequently, the separation membranes may be fitted in the module. Since modification and sterilization can be performed at the same time, more preferably, the aqueous solution containing the hydrophilic polymer and the antioxidant is filled in the module and then the whole module is irradiated with radiation.

Preferably, the substrate may be irradiated with radiation while the substrate is in a wet state with an aqueous solution containing a hydrophilic polymer and an antioxidant. Herein, the wet state refers to a state in which the aqueous solution used for immersing the substrate is removed but the substrate is not dried. Although the water content is not particularly limited, the substrate preferably contains at least one weight percent of water relative to the dry substrate. In other words, the substrate is immersed in the aqueous solution and is then removed from the aqueous solution. Subsequently, the substrate may be irradiated with radiation. Alternatively, the aqueous solution is filled in the module including the substrate and most of the aqueous solution is then discharged from the module with, for example, a nitrogen gas jet. Subsequently, the module may be irradiated with radiation.

In another method, the substrate is immersed in an aqueous solution of a hydrophilic polymer in advance such that the surface of the substrate is coated with the hydrophilic polymer. Subsequently, the substrate may be irradiated with γ-ray while the substrate is immersed in a solution containing an antioxidant. This method can also make the surface of the substrate hydrophilic efficiently.

The area to which the hydrophilic polymer is provided can be variously controlled according to the kind of substrate and the method of modification. For example, in a substrate used as a hollow fiber membrane, an aqueous solution containing a hydrophilic polymer is introduced to the inside of the hollow fiber membrane and the hollow fiber membrane is then irradiated with radiation. In such a case, the hydrophilic polymer can be immobilized on the inner surface of the hollow fiber membrane. For example, this method is preferably applied to an artificial kidney in which the substrate is used such that blood flows only on the inner surface thereof. In addition to the inner surface, when hydrophilization needs to be performed on the outer surface of the hollow fiber membrane, the aqueous solution containing the hydrophilic polymer is brought into contact with the outer surface of the hollow fiber membrane. For example, when hollow fiber membranes are disposed in a module case, the aqueous solution containing the hydrophilic polymer is filled

in the clearance formed between the hollow fiber membranes and the module case.

In a substrate used as a separation membrane, an aqueous solution containing a hydrophilic polymer is filled while the solution is filtered through the membrane. Since the hydrophilic polymer is concentrated on the surface of the membrane, this method is effective at making the surface more hydrophilic. In such a case, when a polymer that does not readily permeate through the membrane, for example, a high-molecular weight hydrophilic polymer, is used as the hydrophilic polymer, the hydrophilic polymer is further concentrated on the surface of the membrane to provide a higher effect.

In contrast, when a low-molecular weight hydrophilic polymer is used, hydrophilization treatment can be performed on the inside of the membrane. For example, in a membrane used for separating biogenic substances and recovering a part of the substances by filtering or dialysis, i.e., a separation membrane of biogenic substances, even when only the surface of the membrane is subjected to hydrophilization, the adsorption of the biogenic substances at the inside of the membrane cannot be suppressed. Accordingly, in an embodiment of the separation membrane of biogenic substances, hydrophilization treatment is preferably performed on the inside of the membrane.

In the present invention, a plurality of substrates are irradiated with radiation at the same time, while a system including the plurality of the substrates is brought into contact with an aqueous solution containing a hydrophilic polymer and an antioxidant. Thus, a plurality of substrates can be modified at one time. In particular, when the plurality of substrates are composed of different materials, this method provides a significant effect. In a known method for modification, it is difficult to modify a plurality of substrates composed of different materials at the same time because the conditions for modifying each substrate significantly depend on the kinds of the substrates.

Herein, the system including a plurality of substrates refers to, for example, a separation membrane system including port elements, separation membranes, and a circuit. For example, modules for blood purification such as an artificial kidney and an adsorption column for exotoxins include a plurality of substrates such as a catheter, a blood circuit, a chamber, an inlet port element and an outlet port element of a module, and separation membranes, the substrates being composed of different materials. In the present invention, all or a part of the substrates can be modified at the same time. Preferably, at least a part of the port elements, the separation membranes, and the circuit is modified. For example, in an artificial kidney system, an inlet port element of a module, an outlet port element of the module, and a blood circuit are connected to a hollow fiber membrane module. An aqueous solution of a hydrophilic polymer is then introduced from the blood circuit to fill the entire system with the solution. Subsequently, the entire system is irradiated with radiation in this state.

Various methods for producing a module for blood purification are known depending on the application. The methods are broadly divided into the steps of producing separation membranes for blood purification and the steps of fitting the separation membranes in the module.

An example of a method for producing a hollow fiber membrane module used in an artificial kidney will now be described. A method for producing a hollow fiber membrane fitted in the artificial kidney includes the following method. A stock solution is prepared by dissolving a polysulfone and polyvinylpyrrolidone in a good solvent or a mixed solvent containing a good solvent. The concentration of the polymer is preferably 10 to 30 weight percent, more preferably, 15 to 25 weight percent. The ratio by weight of the polysulfone to the polyvinylpyrrolidone is preferably 20:1 to 1:5, more preferably, 5:1 to 1:1. N,N-dimethylacetamide, dimethylsulfoxide, dimethylformamide, and N-methylpyrrolidone, and dioxane are preferably used as the good solvent. The stock solution is discharged from an outer tube of a double-annular spinneret to run through a dry step. Subsequently, the stock solution is led to a coagulation bath. An injection liquid or a gas to form a hollow part is discharged from an inner tube of the double-annular spinneret. In this process, the humidity in the dry step affects the characteristics of the membrane. Therefore, moisture may be supplied from the outer surface of the membrane while the stock solution runs through the dry step in order to accelerate a phase separation behavior in the vicinity of the outer surface. As a result, the diameter of the opening is increased. Thus, permeation resistance and diffusion resistance when used for dialysis can be decreased. However, when the relative humidity is excessively high, coagulation of the stock solution at the outer surface becomes dominant. As a result, the diameter of the opening is decreased. Accordingly, permeation resistance and diffusion resistance when used for dialysis are increased. Therefore, the relative humidity is preferably 60% to 90%. In terms of process suitability, the composition of the injection liquid preferably includes the solvent used to prepare the stock solution as a basic component. Regarding the concentration of the injection liquid, for example, when dimethylacetamide is used, an aqueous solution having a concentration of preferably 45 to 80 weight percent, more preferably, 60 to 75 weight percent is used.

Although a method for fitting hollow fiber membranes in a module is not particularly limited, an example of the method is as follows. Firstly, hollow fiber membranes are cut so as to have a desired length. A required number of the hollow fiber membranes are bundled to put in a cylindrical case. Subsequently, both ends are closed with temporal caps. A potting agent is added in both ends of the hollow fiber membranes. Preferably, the potting agent is added while the module is rotated with a centrifuge because the potting agent can be filled uniformly. After the potting agent is solidified, both ends are cut such that both ends of the hollow fiber membranes are opened, thus producing a hollow fiber membrane module.

Fig. 1 shows an example of the basic structure of an artificial kidney system using a hollow fiber membrane module produced by the above method. A bundle of hollow fiber membranes 5 is inserted in a cylindrical plastic case 7. A resin 10 seals both ends of the hollow fibers. The case 7 includes an inlet 8 and an outlet 9 for dialysate. For example, dialysate, physiological saline, or filtered water flows in the outside of the hollow fiber membranes 5. An inlet port element 1 and an outlet port element 2 are disposed at the ends of the case 7. Blood 6 is introduced from a blood inlet 3 disposed in the inlet port element 1, and is introduced to the inside of the hollow fiber membranes 5 by the port element 1 having a funnel shape. The blood 6 filtered with the hollow fiber membranes 5 is collected by the outlet port element 2 to discharge from a blood outlet 4. The blood inlet 3 and the blood outlet 4 are connected to a blood circuit 11.

The present invention will now be described with reference to Examples. Examples 4 to 15 are provided for information only; they do not illustrate a modified substrate as claimed in claim 1.

1. Methods for preparing substrates

Preparation of polysulfone film 1)

Polysulfone (Udel (registered trademark) P-3500 from Teijin Amoco Engineering Plastics Limited) (10 parts by weight) was added to N,N'-dimethylacetamide(80 parts by weight) and allowed to dissolve at room temperature. Thus, a membrane stock solution was prepared. A glass plate was heated with a hot-plate such that the surface temperature of the glass plate was 100°C. The membrane stock solution was cast such that the thickness was 203 μm. The surface temperature was measured with a contact type thermometer. After the casting, the'membrane was left to stand for 5 minutes on the hot-plate to evaporate the solvent. Subsequently, the whole glass plate was immersed in a water bath to prepare a polysulfone film 1. The purpose of the immersion in the water bath is to allow the polysulfone film to be peeled readily from the glass plate.

Preparation of hollow fiber membrane module 1)

Polysulfone (Udel (registered trademark) P-3500 from Teijin Amoco Engineering Plastics Limited) (18 parts by weight) and polyvinylpyrrolidone (K30 from BASF) (9 parts by weight) were added to a mixed solvent containing N,N'-dimethylacetamide (72 parts by weight) and water (1 part by weight). The mixture was heated at 90°C for 14 hours to dissolve the polymers. Thus, a membrane stock solution was prepared. The membrane stock solution was discharged from an outer tube of an orifice type double-cylindrical spinneret having an outer diameter of 0.3 mm and an inner diameter of 0.2 mm. A core liquid containing N,N'-dimethylacetamide (58 parts by weight) and water (42 parts by weight) was discharged from an inner tube. The discharged membrane stock solution was passed through a dry step having a length of 350 mm and was then introduced in a 100% water coagulation bath. Thus, a hollow fiber was prepared.

The resultant 10,000 hollow fibers were inserted in a cylindrical plastic case as shown in Fig. 1, which includes an inlet and an outlet for dialysate. Both ends of the membranes were sealed with a resin to prepare a hollow fiber membrane module 1 for an artificial kidney having an effective membrane area of 1.6 $m^2$.

(Preparation of hollow fiber membrane module 2)

Isotactic-polymethylmethacrylate (5 parts by weight) and syndiotactic-polymethylmethacrylate (20 parts by weight) were added to dimethylsulfoxide (75 parts by weight). The mixture was heated to dissolve the polymers. Thus, a membrane stock solution was prepared. The membrane stock solution was discharged from an outer tube of an orifice type double-cylindrical spinneret. The discharged membrane stock solution was passed through air for 200 mm and was then introduced in a 100% water coagulation bath. Thus, a hollow fiber was prepared. In this process, dry nitrogen was discharged from an inner tube as an inside injection gas. The resultant hollow fiber had an inner diameter of 0.2 mm and a thickness of 0.03 mm. A hollow fiber membrane module 2 having an effective membrane area of 1.6 $m^2$ was prepared using the resultant 10,000 hollow fibers, as in the hollow fiber membrane module 1.

2. Measuring method

(1) Measurement of the soluble hydrophilic polymer ratio

A measurement sample was dried and the dry weight was measured. Subsequently, the sample was dissolved in a solvent that can dissolve both the substrate and the hydrophilic polymer. A solvent that dissolves the hydrophilic polymer but does not dissolve the substrate was added to the resultant solution. As a result of this operation, the substrate and the hydrophilic polymer immobilized on the substrate were precipitated, whereas a soluble hydrophilic polymer remained dissolved. The amount of hydrophilic polymer in the supernatant was quantitatively determined by high performance liquid chromatography (HPLC). Thus, the weight of soluble hydrophilic polymer per unit weight of the measurement sample could be calculated. On the other hand, the elemental analysis of the measurement sample provided the weight of total hydrophilic polymer per unit weight of the measurement sample. The soluble hydrophilic polymer ratio was calculated by dividing the weight of soluble hydrophilic polymer per unit weight of the measurement sample by the weight of total hydrophilic polymer per unit weight of the measurement sample.

When polyvinylpyrrolidone was used as the hydrophilic polymer and Udel (registered trademark) P-3500 was used as the substrate, the soluble hydrophilic polymer ratio was measured as follows. A dry measurement sample was dissolved in N-methyl-2-pyrrolidone such that the concentration of the solution was 2.5 weight percent. Water (1.7 fold by volume) was added dropwise to the solution while the solution was stirred, thereby precipitating the substrate polymer. In this process, the water should not be added at once because the polysulfone is precipitated while the polysulfone becomes entangled with soluble polyvinylpyrrolidone. Attention should be paid because an accurate measurement may be impossible in such a case. The soluble polyvinylpyrrolidone was included in the solution with the dispersed fine polysulfone particles. The solution was filtered with a nonaqueous filter (from Tosoh Corporation, diameter 2.5 μm) for HPLC to remove the fine polysulfone particles in the solution. Subsequently, polyvinylpyrrolidone in the filtrate was quantitatively determined by HPLC under the following conditions.

Apparatus: Waters, GPC-244
Column: TSK-gel GMPWXL, 2 columns
Solvent: Water-based, 0.1 M ammonium chloride, 0.1 N ammonia, pH 9.5
Flow rate: 1.0 mL/min.
Temperature: 23°C

The weight of soluble polyvinylpyrrolidone per unit weight of the measurement sample was calculated from the amount of polyvinylpyrrolidone in the filtrate. This weight was divided by the weight of total polyvinylpyrrolidone per unit weight of the measurement sample, which was determined by elemental analysis. Thus, the soluble polyvinylpyrrolidone ratio was determined.

(2) Dissolution test of hydrophilic polymer

An aqueous solution of a hydrophilic polymer in which a measurement sample was immersed was removed. Subsequently, the measurement sample was immersed in water at 37°C for 4 hours. The volume of water was 0.25 $mL/cm^2$ relative to the area of the surface of the modified substrate. Thus, the amount of dissolved hydrophilic polymer was quantitatively determined.

When the hollow fiber membrane module 1 was used as the measurement sample, the amount of dissolution was measured as follows. The blood side of the hollow fiber membrane module 1 was washed with 700 mL of ultrapure water at room temperature, and the dialysate side thereof was washed with 2,500 mL of ultrapure water at room temperature. The blood side was then washed again with 300 mL of ultrapure water at room temperature to wash away hydrophilic polymers originally included in the filling fluid. Subsequently, the blood side was perfused with 4,000 mL of ultrapure water heated at 37°C for 4 hours at a flow rate of 200 mL/min. Subsequently, the perfusate was concentrated by 200 fold to measure by gel permeation chromatography (GPC). The total amount of hydrophilic polymer dissolved in the perfusate was calculated from the analytical value. When the hydrophilic polymer was polyvinylpyrrolidone, the measurement conditions for GPC were as follows. A GMPWXL column was used, the flow rate was 0.5 mL/min., a mixed solvent of methanol containing 0.1 N lithium nitrate : water = 1 : 1 (volume ratio) was used as the solvent, and the column temperature was 40°C. Polyvinylpyrrolidone K90 (from BASF) was used for a calibration curve of the concentration of polyvinylpyrrolidone.

(3) Measurement of maximum increasing value of ultraviolet absorption value

An ultraviolet absorption value of an aqueous solution of a hydrophilic polymer being in contact with a measurement sample was measured before and after irradiating with radiation. The ultraviolet absorption value was measured in a wavelength range of 260 to 300 nm. An aqueous solution (about 3 mL) for measurement was prepared in a quartz cell having an optical path length of 1 cm. The ultraviolet absorption value was measured with a spectrophotometer U-2000 (from Hitachi, Ltd.) at room temperature. The increasing value of ultraviolet absorption value was calculated by subtracting the ultraviolet absorption value measured before irradiating with radiation from the ultraviolet absorption value measured after irradiating with radiation. The maximum increasing value in the wavelength range of 260 to 300 nm was defined as the maximum increasing value of ultraviolet absorption value.

When a hollow fiber membrane module was used as the measurement sample and an aqueous solution of a hydrophilic polymer was filled in the blood side, after irradiating with radiation, only the aqueous solution dripping by free

fall was sampled. However, when the aqueous solution of the hydrophilic polymer was filled in the blood side, the solution was then discharged by, for example, blowing, and the substrate was irradiated with radiation in a wet state, the aqueous solution might not drip by free fall. In such a case, water is filled in the module again, and the module is left to stand at room temperature for at least one hour. Subsequently, water at the blood side dripping by free fall may be sampled.

When a substrate other than a hollow fiber membrane module is irradiated with radiation in a wet state, the substrate is immersed in water of 0.1 mL/$cm^2$ at room temperature for one hour. Subsequently, the measurement is performed using the water, and the measured value is multiplied by 20. The resultant value is used. In the above hollow fiber membrane module, the volume of filling fluid at the blood side relative to the inner surface area, that is, the bath ratio, is 0.005 mL/$cm^2$. The above calculation indicates that the bath ratio is converted so as to correspond with the above value. If the substrate cannot be immersed in the water volume of 0.1 mL/$cm^2$, water may be appropriately added to perform the measurement. Subsequently, the bath ratio is converted so as to correspond with 0.005 mL/$cm^2$.

(4) Measurement of surface hydrophilic polymer ratio

The hydrophilic polymer ratio on the surface was measured by X-ray photoelectron spectrometry (ESCA). A measurement apparatus ESCALAB220iXL was used and a sample was prepared in the apparatus. In the measurement, the angle of a detector to the angle of incidence of X-ray was 90 degrees. In a film sample, the surface of the film on the glass used for casting was measured. In a hollow fiber membrane sample, the hollow fiber membrane was cut with a single edged knife to form a semicylindrical shape and the inner surface of the hollow fiber membrane was measured. The measurement sample was rinsed with ultrapure water and was then dried at room temperature and at 0.5 Torr for 10 hours. Subsequently, the sample was used for the measurement.

When polyvinylpyrrolidone was used as the hydrophilic polymer and Udel (registered trademark) P-3500 was used as the substrate, the surface polyvinylpyrrolidone ratio was calculated as follows. The amount of nitrogen (a) on the surface and the amount of sulfur (b) on the surface were calculated from the integrated intensity of C1s, N1s, and S2p spectra, which were obtained by ESCA, using a relative sensitivity coefficient provided from the apparatus. The surface polyvinylpyrrolidone ratio was calculated by the following formula:

$$\text{Surface polyvinylpyrrolidone ratio (weight percent)} = a\times100/(a\times111+b\times442)$$

(5) Measurement of immobilization density of polyethylene glycol

A hollow fiber after irradiating with radiation was immersed in distilled water at 37°C for one hour. The volume of the distilled water was 1 L per 1 $m^2$ of the surface area of the substrate. The hollow fiber was washed while distilled water was changed until the amount of polyethylene glycol dissolved into the distilled water was 1 mg or less. Thus, polyethylene glycol that is not immobilized on the substrate was removed. The washed substrate was dried at 50°C and at 0.5 Torr for 10 hours. In a test tube, 10 to 100 mg of the dry substrate was prepared. A mixed solution (2 mL) containing acetic anhydride and para-toluenesulfonic acid was added to the substrate to acetylate the mixture at 120°C for about one hour. After cooling, the wall was washed with 2 mL of purified water. Subsequently, 20% sodium hydrogencarbonate was added to the mixture to neutralize. The neutralized solution was extracted with trichloromethane (5 mL). The extract was analyzed by gas chromatography (hereinafter abbreviated as GC). The analytical conditions for GC were as follows. The amount of polyethylene glycol immobilized on the substrate was determined using a calibration curve prepared in advance.

(Analytical conditions for GC)

Apparatus: Shimadzu GC-9A
Column: Supelcowax-10, 60 m × 0.75 mm I.D.
Carrier gas: Helium
Detector: Flame-ionization detector (FID) ($H_2$ inlet: 0.7 kg/$cm^2$, Air inlet: 0.6 kg/$cm^2$, Temperature: 200°C)
Column temperature: 80°C, holding for 5 min.-(20 min.)-200°C, holding for 5 min.
Injector temperature: 200°C

(6) Measurement of contact angle

The contact angle was measured with a contact angle meter CA-D from Kyowa Interface Science Co., Ltd. The measurement was performed in a room where the room temperature was controlled at 25°C.

(7) Method of adhering test of rabbit blood platelets on film

A film for measurement was disposed on the bottom of a cylindrical polystyrene tube having a diameter of 18 mm. The cylindrical tube was filled with physiological saline. If contaminations, flaws, fold lines, or the like are disposed on the surface of the film, blood platelets are adhered on such areas. Attention should be paid because an accurate evaluation may be impossible in such a case. A blood sample containing an aqueous solution of 3.2% trisodium citrate dihydrate and fresh rabbit blood at a volume ratio of 1:9 was subjected to centrifugal separation at 1,000 rpm for 10 minutes to recover the supernatant (referred to as blood plasma 1). After the supernatant was recovered, the resultant blood was subjected to centrifugal separation again at 3,000 rpm for 10 minutes to recover the supernatant (referred to as blood plasma 2). The blood plasma 1 was diluted by adding the blood plasma 2 (the concentration of blood platelets in the blood plasma 2 was lower than that in the blood plasma 1) to prepare a platelet-rich plasma (hereinafter referred to as PRP) having $20\times10^6$ /mL of blood platelets. The physiological saline prepared in the cylindrical tube was removed and 1.0 mL of the PRP was then added in the cylindrical tube. The cylindrical tube was shaken at 37°C for one hour. Subsequently, the measurement film was washed three times with physiological saline. The blood component was fixed with an aqueous solution of 3% glutaraldehyde. The film was washed with distilled water and was then dried under a reduced pressure for at least 5 hours.

The film was adhered on a specimen support for a scanning electron microscope with a double-sided adhesive tape. A thin film composed of Pt-Pd was deposited on the surface of the film by sputtering to prepare a sample. The surface of the sample was observed with a scanning electron microscope (S800 from Hitachi, Ltd.). Since the blood readily retained in the portions of the film being in contact with the cylindrical tube, the central part of the film was mainly observed at a magnification ratio of 3,000 to count the number of adhered blood platelets found per one field of view ($1.12\times10^3$ $\mu m^2$). The average number of adhered blood platelets in 10 different fields of view in the vicinity of the center of the film was calculated. The number of adhered blood platelets (number/$1.0\times10^3$ $\mu m^2$) was calculated by dividing the above average number of adhered blood platelets by 1.12.

(8) Method of adhering test of human blood platelets on film

A film for measurement was fixed on a polystyrene circular plate having a diameter of 18 mm with a double-sided adhesive tape. If contaminations, flaws, fold lines, or the like are disposed on the surface of the film, blood platelets are adhered on such areas. Attention should be paid because an accurate evaluation may be impossible in such a case. The circular plate was fitted in a Falcon (registered trademark) tube (18 mm in diameter, No. 2051), which was cut in a tubular shape, such that the surface having the film thereon was disposed at the inside of the cylinder. The clearance was filled with Parafilm. The inside of this cylindrical tube was washed with physiological saline and was then filled with physiological saline. Human venous blood was collected and heparin was then added to the blood immediately so as to have a concentration of 50 U/mL. The physiological saline in the cylindrical tube was removed. Subsequently, 1.0 mL of the blood was filled in the cylindrical tube within 10 minutes from the collection. The cylindrical tube was shaken at 37°C for one hour. Subsequently, the measurement film was washed with 10 mL of physiological saline. The blood component was fixed with physiological saline containing 2.5% glutaraldehyde. The film was washed with 20 mL of distilled water. The washed film was then dried at room temperature under a reduced pressure of 0.5 Torr for 10 hours. A thin film composed of Pt-Pd was then deposited on the surface of the film by sputtering to prepare a sample. The surface of the sample was observed with a field emission scanning electron microscope (S800 from Hitachi, Ltd.) at a magnification ratio of 1,500 to count the number of adhered blood platelets found per one field of view ($4.3\times10^3$ $\mu m^2$). The average number of adhered blood platelets in 10 different fields of view in the vicinity of the center of the film was calculated. The average number of adhered blood platelets was defined as the number of adhered blood platelets (number/$4.3\times10^3$ $\mu m^2$).

(9) Method of adhering test of rabbit blood platelets on hollow fiber membrane

Thirty hollow fiber separation membranes were bundled. Both ends of the membranes were fixed in a glass tube module case with an epoxy-based potting agent such that the hollow parts of the hollow fibers were not clogged. Thus, a mini module having a diameter of about 7 mm and a length of about 10 cm was prepared. A blood inlet of the mini module was connected to a dialysate outlet thereof with a silicone tube. In order to wash the hollow fibers and the inside of the module, 100 mL of distilled water was allowed to flow from a blood outlet at a flow rate of 10 mL/min.

Physiological saline was then filled, and a dialysate inlet and the outlet were closed with caps. Subsequently, physiological saline was supplied from the blood inlet at a flow rate of 0.59 mL/min. for two hours to perform priming. A blood sample containing an aqueous solution of 3.2% trisodium citrate dihydrate and fresh rabbit blood at a volume ratio of 1:9 was prepared. Seven milliliters of the blood sample was perfused at a flow rate of 0.59 mL/min. for one hour. Subsequently, the membranes were washed with physiological saline using a 10-mL syringe. An aqueous solution of 3% glutaraldehyde was filled in the inside of the hollow fibers and the dialysate side. The module was left to stand at least one night to perform glutaraldehyde fixation. Subsequently, the glutaraldehyde was washed with distilled water. A hollow fiber membrane was cut out from the mini module and was dried under a reduced pressure for at least 5 hours. The hollow fiber membrane was adhered on a specimen support for a scanning electron microscope with a double-sided adhesive tape. The membrane was then sliced in the longitudinal direction so as to expose the inner surface. A thin film composed of Pt-Pd was deposited on the sample by sputtering. The inner surface of the sample was observed with a scanning electron microscope (S800 from Hitachi, Ltd.) at a magnification ratio of 3,000 to count the number of adhered blood platelets found per one field of view ($1.12\times10^3$ $\mu m^2$). The average number of adhered blood platelets in 10 different fields of view was calculated. The number of adhered blood platelets (number/$1.0\times10^3$ $\mu m^2$) was calculated by dividing the above average number of adhered blood platelets by 1.12.

(10) Method of adhering test of human blood platelets on hollow fiber membrane

A hollow fiber membrane was fixed on a polystyrene circular plate having a diameter of 18 mm with a double-sided adhesive tape. The adhered hollow fiber membrane was cut with a single edged knife to form a semicylindrical shape, thereby exposing the inner surface of the hollow fiber membrane. If contaminations, flaws, fold lines, or the like are disposed on the inner surface of the hollow fiber, blood platelets are adhered on such areas. Attention should be paid because an accurate evaluation may be impossible in such a case. The circular plate was fitted in a Falcon (registered trademark) tube (18 mm in diameter, No. 2051), which was cut in a tubular shape, such that the surface having the hollow fiber membrane thereon was disposed at the inside of the cylinder. The clearance was filled with Parafilm. The inside of this cylindrical tube was washed with physiological saline and was then filled with physiological saline. Human venous blood was collected and heparin was then added to the blood immediately so as to have a concentration of 50 U/mL. The physiological saline in the cylindrical tube was removed. Subsequently, 1.0 mL of the blood was filled in the cylindrical tube within 10 minutes from the collection. The cylindrical tube was shaken at 37°C for one hour. Subsequently, the hollow fiber membrane was washed with 10 mL of physiological saline. The blood component was fixed with physiological saline containing 2.5% glutaraldehyde. The hollow fiber membrane was washed with 20 mL of distilled water. The washed hollow fiber membrane was then dried at room temperature under a reduced pressure of 0.5 Torr for 10 hours. The film was adhered on a specimen support for a scanning electron microscope with a double-sided adhesive tape. A thin film composed of Pt-Pd was then deposited on the surface of the hollow fiber membrane by sputtering to prepare a sample. The inner surface of the hollow fiber membrane was observed with a field emission scanning electron microscope (S800 from Hitachi, Ltd.) at a magnification ratio of 1,500 to count the number of adhered blood platelets found per one field of view ($4.3\times10^3$ $\mu m^2$). The average number of adhered blood platelets in 10 different fields of view in the vicinity of the center of the hollow fiber in the longitudinal direction was calculated. The average number of adhered blood platelets was defined as the number of adhered blood platelets (number/$4.3\times10^3$ $\mu m^2$). This was because the blood readily retained at the end portions of the hollow fiber in the longitudinal direction.

(11) Method of adhering test of human blood platelets in blood circuit for artificial kidney

A blood circuit for an artificial kidney was finely cut into small pieces of about 0.1 g. (If a mesh part was used, the weight was about 0.01 g.) An adhering test of human blood platelets was performed using the small pieces as in the above item (9).

In the adhering tests of blood platelets described in the above items (7) to (11), in order to confirm whether the tests are adequately performed or not, a positive control and a negative control were added in each test as a benchmark. The positive control was a known sample in which a large amount of blood platelets can be adhered. In contrast, the negative control was a known sample in which a small amount of blood platelets is adhered. In the adhering tests of human blood platelets, a sample having a number of adhered blood platelets of at least 40 (/$4.3\times10^3$ $\mu m^2$) under the above experimental conditions was used as the positive control. In addition, a sample having a number of adhered blood platelets of up to 5 (/$4.3\times10^3$ $\mu m^2$) was used as the negative control. In the adhering tests of rabbit blood platelets, a sample having a number of adhered blood platelets of at least 30 (/$1.0\times10^3$ $\mu m^2$) was used as the positive control. In addition, a sample having a number of adhered blood platelets of up to 5 (/$1.0\times10^3$ $\mu m^2$) was used as the negative control. In the following Examples, a hollow fiber membrane used in an artificial kidney Filtryzer BG-1.6U from Toray Industries, Inc. was used as the positive control. A hollow fiber membrane used in an artificial kidney PS-1.6UW from Kawasumi Laboratories, Inc. was used as the negative control. After a test, when the number of blood platelets adhered

on the positive control was the above value or more, and in addition, the number of blood platelets adhered on the negative control was the above value or less, the measurement values could be used. When the number of blood platelets adhered on the controls was not within the above ranges, the test was performed again. In such a case, the freshness of the blood might be insufficient or the blood might be excessively activated.

(12) Adsorption test of IL-6

The same thirty hollow fiber separation membranes as used in the above hollow fiber membrane module 2 were bundled. Both ends of the membranes were fixed in a glass tube module case with an epoxy-based potting agent such that the hollow parts of the hollow fibers were not clogged. Thus, a mini module having a diameter of about 7 mm and a length of about 10 cm was prepared. A blood inlet of the mini module was connected to a dialysate outlet thereof with a silicone tube. In order to wash the hollow fibers and the inside of the module, 100 mL of distilled water was allowed to flow from a blood outlet at a flow rate of 10 mL/min. Subsequently, an aqueous solution of PBS (Dulbecco PBS (-) from Nissui Pharmaceutical Co., Ltd.) was filled, and a dialysate inlet and the outlet were closed with caps.

IL-6 was added to 10 mL of human plasma so as to have a concentration of 1 ng/mL (referred to as liquid 1). The dialysate inlet and the dialysate outlet were closed with the caps, and the inlet of the blood side was connected to the outlet of the blood side with a silicone tube. Perfusion was performed at 37°C for 4 hours with the liquid 1 at a flow rate of 1 mL/min. The IL-6 was quantitatively determined before and after the perfusion. The adsorptivity on the substrate was calculated from the decrease in the IL-6.

(13) Method of adsorptive removal test of oxidized LDL

(a) Preparation of antioxidized LDL antibody

Antioxidized LDL antibody specimens prepared by Itabe et al. (H. Itabe et al., J. Biol. Chem. Vol. 269: p. 15274, 1994) were used. Specifically, mice were immunized by injecting a human atherosclerotic lesion homogenate. The hybridomas were prepared from the spleen cells of the mice, followed by screening those that were allowed to react with LDL that had been treated with copper sulfate. Thus, the antioxidized LDL antibody was prepared. The resultant antibody was classified as mouse IgM, and was not allowed to react with native LDL, acetylated LDL, or malondialdehyde-treated LDL. On the other hand, the antioxidized LDL antibody was allowed to react with some peroxidation products of phosphatidylcholine, including aldehyde derivatives and hydroperoxides of phosphatidylcholine. The antioxidized LDL antibody was dissolved in a 10 mM borate buffer solution (pH 8.5) containing 150 mM NaCl. The solution (protein concentration 0.60 mg/mL) was used as specimens.

(b) Preparation of oxidized LDL

A commercial LDL (from Funakoshi Co., Ltd.) was desalinated and was then diluted with a phosphate buffer solution (hereinafter abbreviated as PBS) so as to have a concentration of 0.2 mg/mL. Subsequently, 2 weight percent of a 0.5 mM aqueous solution of copper sulfate was added to the solution. The solution was allowed to react at 37°C for 5 hours. A 25 mM ethylenediaminetetraacetic acid (EDTA) solution and 10 weight percent sodium azide were added to the resultant solution such that the concentration of the EDTA was 1 weight percent and the concentration of the sodium azide was 0.02 weight percent. This solution was used as an oxidized LDL specimen.

(c) Determination of the concentration of oxidized LDL

The above antioxidized LDL antibody was diluted with PBS so as to have a concentration of 5 μg/mL. The solution was dispensed to a 96-well plate at a rate of 100 μL/well. The plate was shaken at room temperature for two hours. Subsequently the plate was left to stand at 4°C for at least one night to allow the antibody to be adsorbed on the walls.

The antibody solution was removed from the wells. A tris-HCl buffer solution (pH 8.0) containing 1% bovine serum albumin (BSA, Fraction V from Seikagaku Corporation) was dispensed at a rate of 200 μL/well. The plate was shaken at room temperature for two hours to block the walls. The BSA solution was then removed from the wells. Blood plasma containing the oxidized LDL was dispensed at a rate of 100 μL/well. Standard solutions used for plotting a calibration curve were dispensed at a rate of 100 μL/well. The plate was shaken at room temperature for 30 minutes and was then left to stand at 4°C for one night.

The temperature of the specimens was increased to room temperature and the solution was removed from the wells. The wells were washed three times with a tris-HCl buffer solution (pH 8.0) containing 0.05% Tween (registered trademark)-20. A solution of sheep anti-apoB antibody diluted with a 2,000-fold volume of PBS was dispensed in each washed well at a rate of 100 μL/well. The plate was shaken at room temperature for two hours and the anti-apoB antibody

was removed from the wells. The wells were washed three times with a tris-HCl buffer solution (pH 8.0) containing 0.05% Tween-20. Subsequently, alkaline phosphatase-conjugated donkey anti-sheep IgG antibody diluted with a 2,000-fold volume of a tris-HCl buffer solution (pH 8.0) containing 2% Block Ace (from Dainippon Pharmaceutical Co., Ltd.) was dispensed in each washed well at a rate of 100 μL/well. The plate was shaken at room temperature for two hours. Subsequently, the conjugated antibody was removed from the wells. The wells were washed three times with a tris-HCl buffer solution (pH 8.0) containing 0.05% Tween-20. The wells were further washed two times with a tris-HCl buffer solution (pH 8.0). Subsequently, a solution (0.0005 M $MgCl_2$, 1 M diethanolamine buffer solution, pH 9.8) of p-nitrophenyl phosphate (1 mg/mL) was dispensed at a rate of 100 μL/well. The plate was allowed to react at room temperature for an adequate period of time. Subsequently, the absorbance at the wavelength of 415 nm was measured with a plate reader. The calibration curve was plotted using the results with the standard solutions to determine the concentration of the oxidized LDL.

(d) Measurement of adsorptive removal ratio of oxidized LDL

The above oxidized LDL was added to blood plasma of a normal healthy subject (30-years old Japanese, LDL (β lipoprotein) concentration 275 mg/dL, HDL-cholesterol concentration 70 mg/dL) so as to have a concentration of 2 μg/mL.

Seventy hollow fiber membranes were bundled and were inserted in a glass tube module case having a diameter of about 7 mm and a length of 12 cm. Both ends of the hollow fiber membranes were fixed with an epoxy-based potting agent such that the hollow parts of the hollow fiber membranes were not clogged. Thus, a mini module (inner surface area 53 $cm^2$) was prepared. The mini module was washed with ultrapure water at 37°C for 30 minutes. Subsequently a silicone tube (product name ARAM (registered trademark), inner diameter: 0.8 mm, outer diameter: 1 mm, length: 37 cm) was connected to both ends of the mini module through silicone tubes (product name ARAM (registered trademark), inner diameter: 7 mm, outer diameter: 10 mm, length: 2 cm) and irregular shaped connectors. The above blood plasma (1.5 mL) was perfused in the hollow fiber membranes under a nitrogen atmosphere at 25°C for 4 hours with a flow rate of 0.5 mL/min. The volume of blood plasma per 1 $m^2$ of the surface area of hollow fiber membranes was $2.8 \times 10^2$ mL/$m^2$. In addition, the same perfusion procedure was performed for the silicone tubes alone without using the mini module. The concentration of oxidized LDL in the blood plasma was quantitatively determined before and after the perfusion procedure. The adsorptive removal ratio was calculated by the following formulae.

Adsorptive removal ratio of oxidized LDL (%) = adsorptive removal ratio of oxidized LDL (%) in mini module - adsorptive removal ratio of oxidized LDL (%) in silicone tubes alone

Adsorptive removal ratio of oxidized LDL (%) = 100 × (concentration before perfusing - concentration after perfusing) / concentration before perfusing

Examples 1 to 3 illustrate the invention.

(EXAMPLE 1)

The above polysulfone film 1 was used as a substrate. Polyvinylpyrrolidone (K90 from BASF) was used as a hydrophilic polymer and ethanol was used as an antioxidant. The substrate was immersed in an aqueous solution containing the polyvinylpyrrolidone (0.1 weight percent) and ethanol (0.5 weight percent) and was irradiated with γ-ray. The absorbed dose of the γ-ray was 27 kGy. The film was rinsed with purified water. Subsequently, the film was placed in purified water at 80°C and the purified water was stirred for 60 minutes. The purified water was replaced with fresh purified water and was stirred again at 80°C for 60 minutes. Furthermore, the purified water was replaced with fresh purified water and was stirred at 80°C for 60 minutes to remove the adsorbed polyvinylpyrrolidone. The measurement of the surface polyvinylpyrrolidone ratio, the measurement of the contact angle of the surface, the adhering tests of blood

platelets, and the measurement of the soluble hydrophilic polymer ratio were performed using the film. As a result, as shown in Table 1, a polysulfone film having a low soluble hydrophilic polymer ratio, high hydrophilicity, small numbers of adhered blood platelets, and high hematologic compatibility was provided.

(COMPARATIVE EXAMPLE 1)

The above polysulfone film 1 was irradiated with γ-ray in purified water. The absorbed dose of the γ-ray was 28 kGy. The film was rinsed with purified water. Subsequently, the film was placed in purified water at 80°C and the purified water was stirred for 60 minutes. The purified water was replaced with fresh purified water and was stirred again at 80°C for 60 minutes. Furthermore, the purified water was replaced with fresh purified water and was stirred at 80°C for 60 minutes. The measurement of the surface polyvinylpyrrolidone ratio, the measurement of the contact angle of the surface, the adhering tests of blood platelets, and the measurement of the soluble hydrophilic polymer ratio were performed using the film. As a result, as shown in Table 1, the numbers of adhered blood platelets of this film were larger than those of the film in Example 1. Thus, a polysulfone film having low hematologic compatibility was provided.

(COMPARATIVE EXAMPLE 2)

Polyvinylpyrrolidone (K90 from BASF) was used as a hydrophilic polymer and ethanol was used as an antioxidant. The polysulfone film 1 was immersed in an aqueous solution containing the polyvinylpyrrolidone (0.1 weight percent) and ethanol (0.5 weight percent) and was left to stand for three days at room temperature. Subsequently, the film was rinsed with purified water. The film was placed in purified water at 80°C and the purified water was stirred for 60 minutes. The purified water was replaced with fresh purified water and was stirred again at 80°C for 60 minutes. Furthermore, the purified water was replaced with fresh purified water and was stirred at 80°C for 60 minutes. The measurement of the surface polyvinylpyrrolidone ratio, the measurement of the contact angle of the surface, the adhering tests of blood platelets, and the measurement of the soluble hydrophilic polymer ratio were performed using the film. As a result, as shown in Table 1, the contact angle and the numbers of adhered blood platelets of this film were larger than those of the film in Example 1. Thus, a polysulfone film having low hydrophilicity and low hematologic compatibility was provided.

(COMPARATIVE EXAMPLE 3)

The polysulfone film 1 without irradiating with γ-ray was rinsed with purified water. The film was placed in purified water at 80°C and the purified water was stirred for 60 minutes. The purified water was replaced with fresh purified water and was stirred again at 80°C for 60 minutes. Furthermore, the purified water was replaced with fresh purified water and was stirred at 80°C for 60 minutes. The measurement of the surface polyvinylpyrrolidone ratio, the measurement of the contact angle of the surface, the adhering tests of blood platelets, and the measurement of the soluble hydrophilic polymer ratio were performed using the film. As a result, as shown in Table 1, the contact angle and the numbers of adhered blood platelets of this film were larger than those of the film in Example 1. Thus, a polysulfone film having low hydrophilicity and low hematologic compatibility was provided.

Table 1-1

| | Absorbed dose of γ-ray | Hydrophilic polymer | Antioxidant | Surface polyvinylpyrrolidone ratio |
|---|---|---|---|---|
| Example 1 | 27 kGy | Polyvinylpyrrolidone 0.1 wt% | Ethanol 0.5 wt% | 21 wt% |
| Comparative Example 1 | 28 kGy | None | None | < 2 wt% |
| Comparative Example 2 | 0 kGy | Polyvinylpyrrolidone 0.1 wt% | Ethanol 0.5 wt% | 5 wt% |
| Comparative Example 3 | 0 kGy | None | None | < 2 wt% |

Table 1-2

| | Contact angle | Number of adhered human blood platelets (number/ $4.3\times10^3\mu M^2$) | Number of adhered rabbit blood platelets (number/ $10^3\mu m^2$) | Soluble hydrophilic polymer ratio |
|---|---|---|---|---|
| Example 1 | 41° | 0.1 | 0.1 | 0.2 |
| Comparative Example 1 | 43° | 83 | 60 | 0 |
| Comparative Example 2 | 80° | 78 | 50 | 0.1 |
| Comparative Example 3 | 82° | 77 | 58 | 0 |

(EXAMPLE 2)

Polyvinylpyrrolidone (K90 from BASF) was used as a hydrophilic polymer and ethanol was used as an antioxidant. An aqueous solution containing the polyvinylpyrrolidone (0.1 weight percent) and ethanol (0.5 weight percent) was prepared. One thousand milliliters of the aqueous solution was introduced in the blood side and a further 1,000 mL was introduced in the dialysate side of the above hollow fiber membrane module 1 so that the module was filled with the aqueous solution. Subsequently, the module was irradiated with γ-ray. The absorbed dose of the γ-ray was 29 kGy. The dissolution test of polyvinylpyrrolidone was performed using this module. As a result, the amount of dissolution of polyvinylpyrrolidone was 0.15 $mg/m^2$. A hollow fiber in the module was cut into pieces to evaluate the surface polyvinylpyrrolidone ratio, the soluble hydrophilic polymer ratio, and the numbers of adhered blood platelets. Table 2 shows the results.

(EXAMPLE 3)

Polyvinylpyrrolidone (K90 from BASF) was used as a hydrophilic polymer and sodium pyrosulfite was used as an antioxidant. An aqueous solution containing the polyvinylpyrrolidone (0.1 weight percent) and sodium pyrosulfite (500 ppm) was prepared. One thousand milliliters of the aqueous solution was introduced in the blood side and a further 1,000 mL was introduced in the dialysate side of the hollow fiber membrane module 1 so that the module was filled with the aqueous solution. Subsequently, the module was irradiated with γ-ray. The absorbed dose of the γ-ray was 29 kGy. A hollow fiber in the module was cut into pieces to evaluate the surface polyvinylpyrrolidone ratio, the soluble hydrophilic polymer ratio, and the numbers of adhered blood platelets. Table 2 shows the results.

(COMPARATIVE EXAMPLE 4)

One thousand milliliters of purified water was introduced in the blood side and a further 1, 000 mL was introduced in the dialysate side of the hollow fiber membrane module 1 so that the module was filled with the purified water. Subsequently, the module was irradiated with γ-ray. The absorbed dose of the γ-ray was 28 kGy. A hollow fiber in the module was cut into pieces to evaluate the surface polyvinylpyrrolidone ratio, the soluble hydrophilic polymer ratio, and the numbers of adhered blood platelets. As a result, as shown in Table 2, the numbers of adhered blood platelets of this membrane were larger than those of the membranes in Examples 2 and 3. In the filling fluid in the blood side of the module, the maximum increasing value of ultraviolet absorption value in the wavelength range of 260 to 300 nm, the increase being caused by irradiating with γ-ray, was also measured. Furthermore, a mini module was prepared using the same hollow fiber membranes as used in the hollow fiber membrane module 1. The mini module was used for the adsorption test of the oxidized LDL. As shown in Table 3, the adsorptive removal ratio of oxidized LDL of this membrane was lower than that of a hollow fiber membrane on which a cationic hydrophilic polymer was immobilized.

(COMPARATIVE EXAMPLE 5)

Polyvinylpyrrolidone (K90 from BASF) was used as a hydrophilic polymer. An aqueous solution containing the polyvinylpyrrolidone (0.1 weight percent) was prepared. One thousand milliliters of the aqueous solution was introduced in the blood side and a further 1,000 mL was introduced in the dialysate side of the hollow fiber membrane module 1 so that the module was filled with the aqueous solution. Subsequently, the module was irradiated with γ-ray. The absorbed dose of the γ-ray was 29 kGy. A hollow fiber in the module was cut into pieces to evaluate the surface polyvinylpyrrolidone ratio, the soluble hydrophilic polymer ratio, and the numbers of adhered blood platelets. As a result, as shown in Table

2, the numbers of adhered blood platelets of this membrane were larger than those of the membranes in Examples 2 and 3.

(COMPARATIVE EXAMPLE 6)

Ethanol was used as an antioxidant. An aqueous solution containing ethanol (0.5 weight percent) was prepared. One thousand milliliters of the aqueous solution was introduced in the blood side and a further 1,000 mL was introduced in the dialysate side of the hollow fiber membrane module 1 so that the module was filled with the aqueous solution. Subsequently, the module was irradiated with γ-ray. The absorbed dose of the γ-ray was 29 kGy. A hollow fiber in the module was cut into pieces to evaluate the surface polyvinylpyrrolidone ratio, the soluble hydrophilic polymer ratio, and the numbers of adhered blood platelets. As a result, as shown in Table 2, the numbers of adhered blood platelets of this membrane were larger than those of the membranes in Examples 2 and 3.

(COMPARATIVE EXAMPLE 7)

Sodium pyrosulfite was used as an antioxidant. An aqueous solution containing sodium pyrosulfite (500 ppm) was prepared. One thousand milliliters of the aqueous solution was introduced in the blood side and a further 1,000 mL was introduced in the dialysate side of the hollow fiber membrane module 1 so that the module was filled with the aqueous solution. Subsequently, the module was irradiated with γ-ray. The absorbed dose of the γ-ray was 29 kGy. A hollow fiber in the module was cut into pieces to evaluate the surface polyvinylpyrrolidone ratio, the soluble hydrophilic polymer ratio, and the numbers of adhered blood platelets. As a result, as shown in Table 2, the numbers of adhered blood platelets of this membrane were larger than those of the membranes in Examples 2 and 3.

(COMPARATIVE EXAMPLE 8)

Polyvinylpyrrolidone (K90 from BASF) was used as a hydrophilic polymer and ethanol was used as an antioxidant. An aqueous solution containing the polyvinylpyrrolidone (0.1 weight percent) and ethanol (0.5 weight percent) was prepared. One thousand milliliters of the aqueous solution was introduced in the blood side and a further 1,000 mL was introduced in the dialysate side of the hollow fiber membrane module 1 so that the module was filled with the aqueous solution. Subsequently, the module was left to stand for three days at room temperature. The dissolution test of polyvinylpyrrolidone was performed using this module. As a result, the amount of dissolution of polyvinylpyrrolidone was 0.68 $mg/m^2$, which was larger than that of the membrane in Example 2. A hollow fiber in the module was cut into pieces to evaluate the surface polyvinylpyrrolidone ratio, the soluble hydrophilic polymer ratio, and the numbers of adhered blood platelets. Table 2 shows the results. This membrane was not irradiated with γ-ray. Therefore, the numbers of adhered blood platelets were small. However, the amount of dissolution of polyvinylpyrrolidone was large because a grafting reaction or a crosslinking of the polyvinylpyrrolidone was not performed.

Table 2-1

| | Absorbed dose of γ-ray | Hydrophilic polymer | Antioxidant |
|---|---|---|---|
| Example 2 | 29 kGy | Polyvinylpyrrolidone 0.1 wt% | Ethanol 0.5 wt% |
| Example 3 | 29 kGy | Polyvinylpyrrolidone 0.1 wt% | Sodium pyrosulfite 500ppm |
| Comparative Example 4 | 28 kGy | None | None |
| Comparative Example 5 | 29 kGy | Polyvinylpyrrolidone 0.1 wt% | None |
| Comparative Example 6 | 29 kGy | None | Ethanol 0.5 wt% |
| Comparative Example 7 | 29 kGy | None | Sodium pyrosulfite 500ppm |
| Comparative Example 8 | 0 kGy | Polyvinylpyrrolidone 0.1 wt% | Ethanol 0.5 wt% |

Table 2-2

| | Number of adhered human blood platelets (number/$4.3\times10^3\mu m^2$) | Number of adhered rabbit blood platelets (number/$10^3\mu m^3$) | Soluble hydrophilic polymer ratio (%) |
|---|---|---|---|
| Example 2 | 0.1 | 0.1 | 9 |
| Example 3 | 0.1 | 0.1 | 8.5 |

(continued)

| | Number of adhered human blood platelets (number/4.3×$10^3\mu m^2$) | Number of adhered rabbit blood platelets (number/$10^3\mu m^3$) | Soluble hydrophilic polymer ratio (%) |
|---|---|---|---|
| Comparative Example 4 | 65 | 48 | 3.5 |
| Comparative Example 5 | 30 | 25 | 3.6 |
| Comparative Example 6 | 25 | 22 | 9.7 |
| Comparative Example 7 | 31 | 18 | 9.5 |
| Comparative Example 8 | 0.5 | 1 | 73.3 |

(EXAMPLE 4) -REFERENCE ONLY

Polyvinylpyrrolidone (K90 from BASF) was used as a nonionic hydrophilic polymer and polyethyleneimine (weight-average molecular weight: 1,000,000, from BASF) was used as a cationic hydrophilic polymer. An aqueous solution containing the polyvinylpyrrolidone (0.1 weight percent) and the polyethyleneimine (0.1 weight percent) was prepared. One thousand milliliters of the aqueous solution was introduced in the blood side and a further 1,000 mL was introduced in the dialysate side of the hollow fiber membrane module 1 so that the module was filled with the aqueous solution. Subsequently, the module was irradiated with γ-ray. The absorbed dose of the γ-ray was 27 kGy. In the filling fluid in the blood side of the module, the maximum increasing value of ultraviolet absorption value in the wavelength range of 260 to 300 nm, the increase being caused by irradiating with γ-ray, was measured. A hollow fiber in the module was cut into pieces to evaluate the number of adhered blood platelets. Furthermore, a mini module was prepared using the same hollow fiber membranes as used in the hollow fiber membrane module 1. The mini module was used for the adsorption test of the oxidized LDL. Results shown in Table 3 were obtained. Table 3 shows the results.

(EXAMPLE 5) -REFERENCE ONLY

Polyethyleneimine (weight-average molecular weight: 1,000,000, from BASF) was used as a cationic hydrophilic polymer and ethanol was used as an antioxidant. An aqueous solution containing the polyethyleneimine (0.1 weight percent) and ethanol was prepared. One thousand milliliters of the aqueous solution was introduced in the blood side and a further 1, 000 mL was introduced in the dialysate side of the hollow fiber membrane module 1 so that the module was filled with the aqueous solution. Subsequently, the module was irradiated with γ-ray. The absorbed dose of the γ-ray was 29 kGy. In the filling fluid in the blood side of the module, the maximum increasing value of ultraviolet absorption value in the wavelength range of 260 to 300 nm, the increase being caused by irradiating with γ-ray, was measured. As a result, as shown in Table 3, the maximum increasing value of ultraviolet absorption value of this membrane is lower than that of the membrane in Comparative Example 9. A hollow fiber in the module was cut into pieces to evaluate the number of adhered blood platelets. Furthermore, a mini module was prepared using the same hollow fiber membranes as used in the hollow fiber membrane module 1. The mini module was used for the adsorption test of the oxidized LDL. Table 3 shows the results.

(COMPARATIVE EXAMPLE 9)

Polyethyleneimine (weight-average molecular weight: 1,000,000, from BASF) was used as a cationic hydrophilic polymer. An aqueous solution containing the polyethyleneimine (0.1 weight percent) was prepared. One thousand milliliters of the aqueous solution was introduced in the blood side and a further 1,000 mL was introduced in the dialysate side of the hollow fiber membrane module 1 so that the module was filled with the aqueous solution. Subsequently, the module was irradiated with γ-ray. The absorbed dose of the γ-ray was 28 kGy. In the filling fluid in the blood side of the module, the maximum increasing value of ultraviolet absorption value in the wavelength range of 260 to 300 nm, the increase being caused by irradiating with γ-ray, was measured. A hollow fiber in the module was cut into pieces to evaluate the number of adhered blood platelets. Furthermore, a mini module was prepared using the same hollow fiber membranes as used in the hollow fiber membrane module 1. The mini module was used for the adsorption test of the

oxidized LDL. As a result, as shown in Table 3, the number of adhered blood platelets of this membrane was larger than that of the membrane in Example 4.

Table 3-1

| | Absorbed dose of γ-ray | Nonionic hydrophilic polymer | Cationic hydrophilic polymer | Antioxidant |
|---|---|---|---|---|
| Example 4 | 27 kGy | Polyvinylpyrrolidone 0.1 wt% | Polyethyleneimine 0.1 wt% | None |
| Example 5 | 29 kGy | None | Polyethyleneimine 0.1 wt% | Ethanol 0.5 wt% |
| Comparative Example 9 | 28 kGy | None | Polyethyleneimine 0.1 wt% | None |
| Comparative Example 4 | 28 kGy | None | None | None |

Table 3-2

| | Number of adhered human blood platelets (number/ $4.3\times10^3\mu m^2$) | Soluble hydrophilic polymer ratio (%) | Maximum increasing value of ultraviolet absorption value | Adsorptive removal ratio of oxidized LDL (%) |
|---|---|---|---|---|
| Example 4 | 0.2 | 10 | 0.60 | 26 |
| Example 5 | 12 | 12 | 0.25 | 27 |
| Comparative Example 9 | 14 | 8.7 | 0.61 | 30 |
| Comparative Example 4 | 65 | 3.5 | 0.15 | 10 |

(EXAMPLE 6) - REFERENCE ONLY

Five thousand milliliters of ultrapure water at 40°C was introduced in the blood side and a further 5,000 mL of ultrapure water at 40°C was introduced in the dialysate side of the above hollow fiber membrane module 2 in order to wash the module. Polyethylene glycol (Macrogol (registered trademark) 6000 from NOF Corporation) was used as a hydrophilic polymer. An aqueous solution containing the polyethylene glycol (0.075 weight percent) was prepared. One thousand milliliters of the aqueous solution was introduced in the blood side and a further 1,000 mL was introduced in the dialysate side of the module so that the module was filled with the aqueous solution. Subsequently, the module was irradiated with γ-ray. The absorbed dose of the γ-ray was 28 kGy. The measurement of the immobilization density of polyethylene glycol, the adhering test of blood platelets, and the adsorption test of IL-6 were performed with the module. Table 4 shows the results.

(EXAMPLE 7) - REFERENCE ONLY

Five thousand milliliters of ultrapure water at 40°C was introduced in the blood side and a further 5,000 mL of ultrapure water at 40°C was introduced in the dialysate side of the hollow fiber membrane module 2 in order to wash the module. Polyethylene glycol (Macrogol (registered trademark) 6000 from NOF Corporation) was used as a hydrophilic polymer. An aqueous solution containing the polyethylene glycol (0.100 weight percent) was prepared. One thousand milliliters of the aqueous solution was introduced in the blood side and a further 1,000 mL was introduced in the dialysate side of the module so that the module was filled with the aqueous solution. Subsequently, the module was irradiated with γ-ray. The absorbed dose of the γ-ray was 28 kGy. The measurement of the immobilization density of polyethylene glycol, the adhering test of blood platelets, and the adsorption test of IL-6 were performed with the module. Table 4 shows the results.

(EXAMPLE 8) -REFERENCE ONLY

Five thousand milliliters of ultrapure water at 40°C was introduced in the blood side and a further 5,000 mL of ultrapure water at 40°C was introduced in the dialysate side of the hollow fiber membrane module 2 in order to wash the module. Polyvinylpyrrolidone (K90 from ISP) was used as a hydrophilic polymer. An aqueous solution containing the polyvinylpyrrolidone (0.100 weight percent) was prepared. One thousand milliliters of the aqueous solution was introduced in the blood side and a further 1,000 mL was introduced in the dialysate side of the module so that the module was filled with the aqueous solution. Subsequently, the module was irradiated with γ-ray. The absorbed dose of the γ-ray was 28 kGy. The adhering test of blood platelets and the adsorption test of IL-6 were performed with the module. Table 4 shows the results.

(COMPARATIVE EXAMPLE 10)

Five thousand milliliters of ultrapure water at 40°C was introduced in the blood side and a further 5,000 mL of ultrapure water at 40°C was introduced in the dialysate side of the hollow fiber membrane module 2 in order to wash the module. Polyethylene glycol (Macrogol (registered trademark) 6000 from NOF Corporation) was used as a hydrophilic polymer. An aqueous solution containing the polyethylene glycol (0.010 weight percent) was prepared. One thousand milliliters of the aqueous solution was introduced in the blood side and a further 1, 000 mL was introduced in the dialysate side of the module so that the module was filled with the aqueous solution. Subsequently, the module was irradiated with γ-ray. The absorbed dose of the γ-ray was 28 kGy. The measurement of the immobilization density of polyethylene glycol, the adhering test of blood platelets, and the adsorption test of IL-6 were performed with the module. Table 4 shows the results.

(COMPARATIVE EXAMPLE 11)

Five thousand milliliters of ultrapure water at 40°C was introduced in the blood side and a further 5,000 mL of ultrapure water at 40°C was introduced in the dialysate side of the hollow fiber membrane module 2 in order to wash the module. Polyethylene glycol (polyethylene glycol #200 from Nacalai Tesque, Inc.) was used as a hydrophilic polymer. An aqueous solution containing the polyethylene glycol (0.100 weight percent) was prepared. One thousand milliliters of the aqueous solution was introduced in the blood side and a further 1,000 mL was introduced in the dialysate side of the module so that the module was filled with the aqueous solution. Subsequently, the module was irradiated with γ-ray. The absorbed dose of the γ-ray was 28 kGy. The measurement of the immobilization density of polyethylene glycol, the adhering test of blood platelets, and the adsorption test of IL-6 were performed with the module. Table 4 shows the results.

(COMPARATIVE EXAMPLE 12)

Five thousand milliliters of ultrapure water at 40°C was introduced in the blood side and a further 5,000 mL of ultrapure water at 40°C was introduced in the dialysate side of the hollow fiber membrane module 2 in order to wash the module. Polyethylene glycol (Mw 900,000 from Scientific Polymers Products, Inc.) was used as a hydrophilic polymer. An aqueous solution containing the polyethylene glycol (0.100 weight percent) was prepared. One thousand milliliters of the aqueous solution was introduced in the blood side and a further 1,000 mL was introduced in the dialysate side of the module so that the module was filled with the aqueous solution. Subsequently, the module was irradiated with γ-ray. The absorbed dose of the γ-ray was 28 kGy. The measurement of the immobilization density of polyethylene glycol, the adhering test of blood platelets, and the adsorption test of IL-6 were performed with the module. Table 4 shows the results.

(COMPARATIVE EXAMPLE 13)

Five thousand milliliters of ultrapure water at 40°C was introduced in the blood side and a further 5,000 mL of ultrapure water at 40°C was introduced in the dialysate side of the hollow fiber membrane module 2 in order to wash the module. Subsequently, the module was filled with ultrapure water and was irradiated with γ-ray. The absorbed dose of the γ-ray was 28 kGy. The adhering test of blood platelets and the adsorption test of IL-6 were performed with the module. Table 4 shows the results.

Table 4

| | Number of adhered human blood platelets (number/$4.3\times10^3\mu m^2$) | Adsorptivity to IL-6 ($ng/cm^2$) | Immobilization density of polyethylene glycol ($mg/m^2$) |
|---|---|---|---|
| Example 6 | 0.56 | 0.209 | 384 |

(continued)

| | Number of adhered human blood platelets (number/$4.3\times10^3\mu m^2$) | Adsorptivity to IL-6 ($ng/cm^2$) | Immobilization density of polyethylene glycol ($mg/m^2$) |
|---|---|---|---|
| Example 7 | 0.43 | 0.180 | 353 |
| Example 8 | 0.99 | 0.163 | - |
| Comparative Example 10 | 3.23 | 0.032 | 137 |
| Comparative Example 11 | 48.59 | 0.282 | 172 |
| Comparative Example 12 | 0.56 | 0.053 | 239 |
| Comparative Example 13 | 100 or more | 0.162 | 0 |

(EXAMPLE 9) -REFERENCE ONLY

A connector part at the blood side of an artificial kidney module of a commercial blood circuit for an artificial kidney (artificial kidney blood circuit H-102-KTS from Toray Medical Co., Ltd) was cut into small pieces to prepare a measurement sample of 1 g. Polyvinylpyrrolidone (K90 from ISP) was used as a hydrophilic polymer and ethanol was used as an antioxidant. The measurement sample was immersed in an aqueous solution (60 mL) containing the polyvinylpyrrolidone (0.100 weight percent) and ethanol (0.100 weight percent), and was irradiated with γ-ray. The adhering test of blood platelets was performed. Table 5 shows the result.

(EXAMPLE 10) - REFERENCE ONLY

A blood tube part of a commercial blood circuit for an artificial kidney (artificial kidney blood circuit H-102-KTS from Toray Medical Co., Ltd) was cut into small pieces to prepare a measurement sample of 1 g. Polyvinylpyrrolidone (K90 from ISP) was used as a hydrophilic polymer and ethanol was used as an antioxidant. The measurement sample was immersed in an aqueous solution (60 mL) containing the polyvinylpyrrolidone (0.100 weight percent) and ethanol (0.100 weight percent), and was irradiated with γ-ray. The adhering test of blood platelets was performed. Table 5 shows the result.

(EXAMPLE 11) - REFERENCE ONLY

A blood chamber part of a commercial blood circuit for an artificial kidney (artificial kidney blood circuit H-102-KTS from Toray Medical Co., Ltd) was cut into small pieces to prepare a measurement sample of 1 g. Polyvinylpyrrolidone (K90 from ISP) was used as a hydrophilic polymer and ethanol was used as an antioxidant. The measurement sample was immersed in an aqueous solution (60 mL) containing the polyvinylpyrrolidone (0.100 weight percent) and ethanol (0.100 weight percent), and was irradiated with γ-ray. The adhering test of blood platelets was performed. Table 5 shows the result.

(EXAMPLE 12) - REFERENCE ONLY

A mesh part of a commercial blood circuit for an artificial kidney (artificial kidney blood circuit H-102-KTS from Toray Medical Co., Ltd) was cut into small pieces to prepare a measurement sample of 1 g. Polyvinylpyrrolidone (K90 from ISP) was used as a hydrophilic polymer and ethanol was used as an antioxidant. The measurement sample was immersed in an aqueous solution (60 mL) containing the polyvinylpyrrolidone (0.100 weight percent) and ethanol (0.100 weight percent), and was irradiated with γ-ray. The adhering test of blood platelets was performed. Table 5 shows the result.

(COMPARATIVE EXAMPLE 14)

A connector part at the blood side of an artificial kidney module of a commercial blood circuit for an artificial kidney (artificial kidney blood circuit H-102-KTS from Toray Medical Co., Ltd) was cut into small pieces to perform the

adhering test of blood platelets. Table 5 shows the result.

(COMPARATIVE EXAMPLE 15)

A blood tube part of a commercial blood circuit for an artificial kidney (artificial kidney blood circuit H-102-KTS from Toray Medical Co., Ltd) was cut into small pieces to perform the adhering test of blood platelets. Table 5 shows the result.

(COMPARATIVE EXAMPLE 16)

A blood chamber part of a commercial blood circuit for an artificial kidney (artificial kidney blood circuit H-102-KTS from Toray Medical Co., Ltd) was cut into small pieces to perform the adhering test of blood platelets. As a result, as shown in Table 5, the number of adhered blood platelets was 7.0 (/$4.3\times10^3$ $\mu m^2$).

(COMPARATIVE EXAMPLE 17)

A mesh part of a commercial blood circuit for an artificial kidney (artificial kidney blood circuit H-102-KTS from Toray Medical Co., Ltd) was cut into small pieces to perform the adhering test of blood platelets. Table 5 shows the result.

Table 5

| | Number of adhered human blood platelets(number/$4.3\times10^3$ $\mu m^2$) |
|---|---|
| Example 9 | 0.67 |
| Example 10 | 0.67 |
| Example 11 | 0.33 |
| Example 12 | 29.00 |
| Comparative Example 14 | 5.67 |
| Comparative Example 15 | 3.33 |
| Comparative Example 16 | 7.00 |
| Comparative Example 17 | 100 or more |

(EXAMPLE 13) - REFERENCE ONLY

A commercial glassy carbon plate (from Toyo Tanso Co., Ltd.) was used as a substrate. Polyvinylpyrrolidone (K90 from BASF) was used as a hydrophilic polymer and ethanol was used as an antioxidant. The substrate was immersed in an aqueous solution containing the polyvinylpyrrolidone (0.01 weight percent) and ethanol (0.1 weight percent) and was irradiated with $\gamma$-ray. The absorbed dose of the $\gamma$-ray was 27 kGy. The film was rinsed with purified water. Subsequently, the film was placed in purified water at 80°C and the purified water was stirred for 60 minutes. The purified water was replaced with fresh purified water and was stirred again at 80°C for 60 minutes. Furthermore, the purified water was replaced with fresh purified water and was stirred at 80°C for 60 minutes to remove the adsorbed polyvinylpyrrolidone. The contact angle of the surface of the film was measured. The contact angle of the film was 39 degrees, whereas that of an untreated film was 98 degrees. This result showed that the film was significantly subjected to hydrophilization.

(EXAMPLE 14) - REFERENCE ONLY

A commercial glassy carbon plate (from Toyo Tanso Co., Ltd.) was used as a substrate. Polyvinylpyrrolidone (K90 from BASF) was used as a hydrophilic polymer. The substrate was immersed in an aqueous solution containing the polyvinylpyrrolidone (0.01 weight percent) and was irradiated with $\gamma$-ray. The absorbed dose of the $\gamma$-ray was 27 kGy. The film was rinsed with purified water. Subsequently, the film was placed in purified water at 80°C and the purified water was stirred for 60 minutes. The purified water was replaced with fresh purified water and was stirred again at 80°C for 60 minutes. Furthermore, the purified water was replaced with fresh purified water and was stirred at 80°C for 60 minutes to remove the adsorbed polyvinylpyrrolidone. The contact angle of the surface of the film was measured. The contact angle of the film was 52 degrees, whereas that of the untreated film was 98 degrees. This result showed that

the film was significantly subjected to hydrophilization.

(COMPARATIVE EXAMPLE 18)

The glassy carbon plate used in Example 13 was irradiated with γ-ray in purified water. The absorbed dose of the γ-ray was 28 kGy. The film was rinsed with purified water. Subsequently, the film was placed in purified water at 80°C and the purified water was stirred for 60 minutes. The purified water was replaced with fresh purified water and was stirred again at 80°C for 60 minutes. Furthermore, the purified water was replaced with fresh purified water and was stirred at 80°C for 60 minutes. The contact angle of the surface of the film was 98 degrees, which was the same as the 98 degrees of the untreated film.

(EXAMPLE 15) -REFERENCE ONLY

A commercial carbon sheet (from Toray Industries, Inc.) was used as a substrate. Polyvinylpyrrolidone (K90 from BASF) was used as a hydrophilic polymer and ethanol was used as an antioxidant. The substrate was immersed in an aqueous solution containing the polyvinylpyrrolidone (0.1 weight percent) and ethanol (0.1 weight percent) and was irradiated with γ-ray. The absorbed dose of the γ-ray was 27 kGy. The film was rinsed with purified water. Subsequently, the film was placed in purified water at 80°C and the purified water was stirred for 60 minutes. The purified water was replaced with fresh purified water and was stirred again at 80°C for 60 minutes. Furthermore, the purified water was replaced with fresh purified water and was stirred at 80°C for 60 minutes to remove the adsorbed polyvinylpyrrolidone. The contact angle of the surface of the film was measured. The contact angle of the film was 30 degrees, whereas that of an untreated film was 131 degrees. This result showed that the film was significantly subjected to hydrophilization.

Industrial Applicability

According to a modified substrate of the present invention, a hydrophilic polymer is immobilized on the surface, and in addition, excessive crosslinking, degradation or the like of the hydrophilic polymer is prevented. Accordingly, the adhesion of organic matter such as proteins, or biogenic substances can be suppressed. In particular, the modified substrate of the present invention has high hematologic compatibility. Furthermore, the high hematologic compatibility can be achieved while the adsorption of a cytokine is maintained.

The modified substrate of the present invention can be widely used for applications that require hydrophilicity on the surface. For example, the modified substrate of the present invention can be preferably used in medical devices such as an artificial blood vessel, a catheter, a blood bag, a blood filter, a contact lens, an intraocular lens, an artificial kidney, an artificial lung, and auxiliary instruments for surgical operation. The modified substrate of the present invention can be preferably used in separation membranes of biogenic substances such as amino acids, peptides, saccharides, proteins, and composites thereof. The modified substrate of the present invention can be preferably used in instruments used for biological experiments such as pipette tips, tubes, Petri dishes, and sample collection tubes; bioreactors; molecular motors; DDS; protein chips; DNA chips; biosensors; and components of analytical instruments such as an atomic force microscope (AFM), a scanning near-field optical microscope (SNOM), and a surface plasmon resonance (SPR) sensor. In addition, the modified substrate of the present invention can be preferably used in separation membranes for water treatment such as membranes for a water purifier, membranes for purifying clean water, membranes for purifying sewage, and reverse osmosis (RO) membranes. In particular, the modified substrate of the present invention is preferably used for applications in which the substrate is brought into contact with a biogenic substance, for example, a module for blood purification such as an artificial kidney.

## Claims

1. A modified substrate comprising a hydrophilic polymer, wherein the substrate is composed of a polymeric material which is a polysulfone or copolymers thereof and the hydrophilic polymer which is a polyvinylpyrrolidone or a polyvinylpyrrolidone copolymer, wherein the soluble hydrophilic polymer ratio, measured as described in the specification, is 15 weight percent or less and a number of adhered human blood platelets adhered in an area of $4.3 \times 10^3$ μ$m^2$, measured as described in the specification, is 10 or less, and the surface hydrophilic polymer ratio, measured as described in the specification, is at least 20 weight percent, the number of adhered blood platelets being defined as the number of blood platelets adhered on the surface of the modified substrate when it is brought into contact with blood for one hour, and
wherein the substrate is obtainable by irradiating with radiation with an absorbed dose of at least 20kGy while the

substrate is brought into contact with an aqueous solution of the hydrophilic polymer and antioxidant selected from an alcohol, saccharide or inorganic salt.

2. The modified substrate according to claim 1, wherein the antioxidant is selected from methanol, ethanol, propanol, ethylene glycol, glycerine, glucose, galactose, mannose, trehalose, sodium hydrosulfite, sodium pyrosulfite, and sodium dithionate.

3. The modified substrate according to claim 1 or 2, wherein the amount of dissolution of the hydrophilic polymer is 0.5 mg/$m^2$ or less, wherein the amount of dissolution of the hydrophilic polymer is the amount of the hydrophilic polymer that is dissolved into purified water when the modified substrate is brought into contact with the purified water at 37°C for 4 hours, when measured as described in the specification.

4. A medical device comprising the modified substrate according to any one of claims 1 to 3.

5. A separation membrane comprising the modified substrate according to any one of claims 1 to 3.

6. The separation membrane according to claim 5, wherein the separation membrane is a hollow fiber membrane.

7. The separation membrane according to claim 6, wherein the hydrophilic polymer is bonded on the inner surface of the hollow fiber membrane.

8. The separation membrane according to claim 7, wherein the hydrophilic polymer is further bonded on the inside of the hollow fiber membrane.

9. The separation membrane according to claim 6, wherein the hollow fiber membrane is obtained from a stock solution prepared by dissolving a polysulfone and polyvinylpyrrolidone.

10. The separation membrane according to claim 9, wherein the ratio by weight of the polysulfone to the polyvinylpyrrolidone in the stock solution is 20:1 to 1:5.

11. A medical device comprising the separation membrane according to any one of claims 5 to 10.

**Patentansprüche**

1. Modifiziertes Substrat, umfassend ein hydrophiles Polymer, wobei das Substrat aus einem Polymermaterial, das Polysulfon oder Copolymere davon ist, und dem hydrophilen Polymer, das ein Polyvinylpyrrolidon oder ein Polyvinylpyrrolidon-Copolymer ist, zusammengesetzt ist, wobei der wie in der Beschreibung beschrieben gemessene Anteil an löslichen hydrophilen Polymeren 15 Gew.-% oder weniger beträgt und die wie in der Beschreibung beschrieben gemessene Anzahl an anhaftenden menschlichen Blutplättchen, die auf einer Fläche von 4,3 x $10^3$ $\mu m^2$ anhaften, 10 oder weniger beträgt, und der wie in der Beschreibung beschrieben gemessene Anteil an hydrophilen Polymeren an der Oberfläche zumindest 20 Gew.-% beträgt; wobei die Anzahl an anhaftenden Blutplättchen als die Anzahl an Blutplättchen definiert ist, die an der Oberfläche des modifizierten Substrats anhaften, wenn dieses eine Stunde lang mit Blut in Kontakt gebracht wird, und wobei das Substrat erhältlich ist, indem es mit Bestrahlung mit einer absorbierten Strahlendosis von zumindest 20 kGy bestrahlt wird, während das Substrat mit einer wässrigen Lösung des hydrophilen Polymers und eines Antioxidans, das aus einem Alkohol, einem Saccharid oder einem anorganischen Salz ausgewählt ist, in Kontakt gebracht wird.

2. Modifiziertes Substrat nach Anspruch 1, wobei das Antioxidans aus Methanol, Ethanol, Propanol, Ethylenglykol, Glycerin, Glucose, Galactose, Mannose, Trehalose, Natriumhydrosulfit, Natriumpyrosulfit und Natriumdithionat ausgewählt ist.

3. Modifiziertes Substrat nach Anspruch 1 oder 2, wobei die gelöste Menge des hydrophilen Polymers 0,5 mg/$m^2$ oder weniger beträgt, wobei die gelöste Menge des hydrophilen Polymers jene wie in der Beschreibung beschrieben gemessene Menge an hydrophilem Polymer ist, die sich in gereinigtem Wasser löst, wenn das modifizierte Substrat bei 37 °C 4 h lang mit dem gereinigten Wasser in Kontakt gebracht wird.

4. Medizinische Vorrichtung, die ein modifiziertes Substrat nach einem der Ansprüche 1 bis 3 umfasst.

5. Trennmembran, die ein modifiziertes Substrat nach einem der Ansprüche 1 bis 3 umfasst.

6. Trennmembran nach Anspruch 5, wobei die Trennmembran eine Hohlfasermembran ist.

7. Trennmembran nach Anspruch 6, wobei das hydrophile Polymer an die Innenfläche der Hohlfasermembran gebunden ist.

8. Trennmembran nach Anspruch 7, wobei das hydrophile Polymer weiters an das Innere der Hohlfasermembran gebunden ist.

9. Trennmembran nach Anspruch 6, wobei die Hohlfasermembran aus einer Stammlösung erhalten wird, die durch Lösen eines Polysulfons und Polyvinylpyrrolidons hergestellt wird.

10. Trennmembran nach Anspruch 9, wobei das Gewichtsverhältnis zwischen Polysulfon und Polyvinylpyrrolidon in der Stammlösung 20:1 bis 1:5 beträgt.

11. Medizinische Vorrichtung, die eine Trennmembran nach einem der Ansprüche 5 bis 10 umfasst.

## Revendications

1. Substrat modifié comprenant un polymère hydrophile, dans lequel le substrat est composé d'un matériau polymère qui est une polysulfone ou des copolymères de celle-ci et du polymère hydrophile qui est une polyvinylpyrrolidone ou un copolymère de polyvinylpyrrolidone, dans lequel le rapport du polymère hydrophile soluble, mesuré tel que décrit dans le mémoire descriptif, est de 15 pour cent en poids ou moins et un nombre de plaquettes sanguines humaines fixées, fixées sur une aire de 4.3 x $10^3$ $\mu m^2$, mesuré tel que décrit dans le mémoire descriptif, est de 10 ou moins, et le rapport du polymère hydrophile de surface, mesuré tel que décrit dans le mémoire descriptif, est d'au moins 20 pour cent en poids, le nombre de plaquettes sanguines fixées étant défini comme étant le nombre de plaquettes sanguines fixées sur la surface du substrat modifié lorsqu'il est amené en contact avec du sang pendant une heure, et
dans lequel le substrat peut être obtenu par irradiation avec un rayonnement à une dose absorbée d'au moins 20 kGy tandis que le substrat est amené en contact avec une solution aqueuse du polymère hydrophile et d'un antioxydant choisi parmi un alcool, un saccharide ou un sel inorganique.

2. Substrat modifié selon la revendication 1, dans lequel l'antioxydant est choisi parmi le méthanol, l'éthanol, le propanol, l'éthylène glycol, la glycérine, le glucose, le galactose, le mannose, le tréhalose, l'hydrosulfite de sodium, le pyrosulfite de sodium et le dithionate de sodium.

3. Substrat modifié selon la revendication 1 ou 2, dans lequel la quantité de dissolution du polymère hydrophile est de 0,5 mg/$m^2$ ou moins, dans lequel la quantité de dissolution du polymère hydrophile est la quantité du polymère hydrophile qui est dissoute dans de l'eau purifiée lorsque le substrat modifié est amené en contact avec l'eau purifiée à 37 °C pendant 4 heures, lorsqu'elle est mesurée tel que décrit dans le mémoire descriptif.

4. Dispositif médical comprenant le substrat modifié selon l'une quelconque des revendications 1 à 3.

5. Membrane de séparation comprenant le substrat modifié selon l'une quelconque des revendications 1 à 3.

6. Membrane de séparation selon la revendication 5, dans laquelle la membrane de séparation est une membrane à fibres creuses.

7. Membrane de séparation selon la revendication 6, dans laquelle le polymère hydrophile est lié sur la surface interne de la membrane à fibres creuses.

8. Membrane de séparation selon la revendication 7, dans laquelle le polymère hydrophile est en outre lié sur l'intérieur de la membrane à fibres creuses.

9. Membrane de séparation selon la revendication 6, dans laquelle la membrane à fibres creuses est obtenue à partir d'une solution de base préparée par dissolution d'une polysulfone et d'une polyvinylpyrrolidone.

**10.** Membrane de séparation selon la revendication 9, dans laquelle le rapport en poids de la polysulfone à la polyvinylpyrrolidone dans la solution de base est de 20/1 à 1/5.

**11.** Dispositif médical comprenant la membrane de séparation selon l'une quelconque des revendications 5 à 10.

Fig. 1

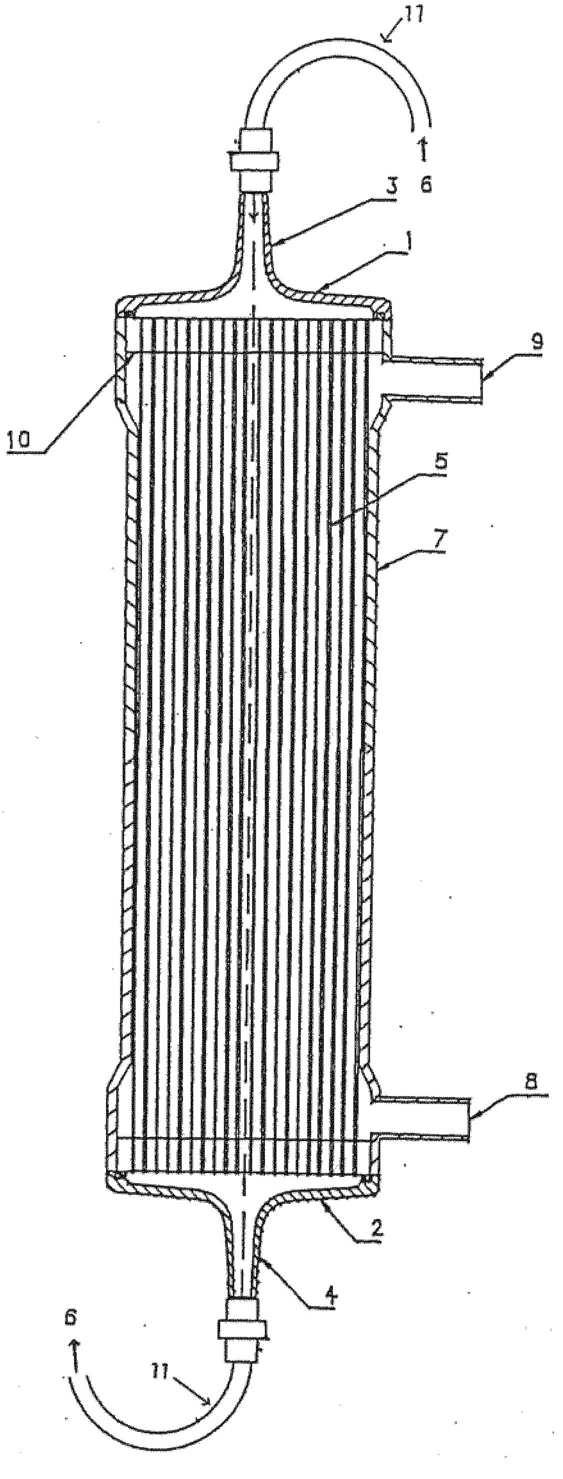

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 10118472 A
- JP 6238139 A
- JP H11047570 A
- JP 2000254222 A
- JP 2000237557 A
- JP 53134876 A
- EP 1439212 A


### Non-patent literature cited in the description


- **H. ITABE et al.** *J. Biol. Chem.,* 1994, vol. 269, 15274